# EUROPÄISCHE PATENTSCHRIFT

(11) **EP 2 373 617 B1**
(45) Veröffentlichungstag und Bekanntmachung des Hinweises auf die Patenterteilung: **25.02.2015**
(21) Anmeldenummer: 09756746.5
(22) Anmeldetag: 24.11.2009
(51) Int. Cl.: C07D 207/267, B01D 53/14, C07C 57/13, C09D 9/00, C11D 7/50

(54) **MISCHUNGEN AUS ITACONSÄURE ODER ITACONSÄUREDERIVATEN UND PRIMÄREN AMINEN ZUR HERSTELLUNG VON 1,3- UND 1,4-ALKYLMETHYLPYRROLIDONEN**
MIXTURES OF ITACONIC ACID OR ITACONIC ACID DERIVATIVES AND PRIMARY AMINES FOR PRODUCING 1,3- AND 1,4-ALKYL METHYL PYRROLIDONES
MÉLANGES D'ACIDE ITACONIQUE OU DE DÉRIVÉS D'ACIDE ITACONIQUE ET D'AMINES PRIMAIRES POUR LA PRODUCTION DE 1,3- ET DE 1,4-ALKYLMÉTHYLPYRROLIDONES

(30) Priorität: 04.12.2008 EP 08170701
(43) Veröffentlichungstag der Anmeldung: 12.10.2011
(73) Patentinhaber: BASF SE, 67056 Ludwigshafen (DE)
(72) Erfinder: WABNITZ, Tobias, 68169 Mannheim (DE); PINKOS, Rolf, 67098 Bad Dürkheim (DE); OTT, Karl, 68723 Plankstadt (DE); LAMM, Katja, 68165 Mannheim (DE)
(86) Internationale Anmeldenummer: PCT/EP2009/065751
(87) Internationale Veröffentlichungsnummer: WO 2010/063617

(56) Entgegenhaltungen:
- EP-A1- 1 864 971
- WO-A1-02/04548
- WO-A1-99/65483
- WO-A2-2007/118185
- CN-A- 1 609 089
- DE-A1- 1 620 191
- FR-E- 89 573
- US-A- 2 604 449
- AKASHI, HIROYOSHI: "Chemistry and application of itaconic acid. VI. Syntheses of N-substituted itaconimides and their copolymerization" KOGYO KAGAKU ZASSHI , 65, 982-5 CODEN: KGKZA7; ISSN: 0368-5462, 1962, XP009129151
- URZUA, M. ET AL: "N-1-alkylitaconamic acids-co-styrene copolymers. 1. Synthesis, characterization and monomer reactivity ratios" JOURNAL OF MACROMOLECULAR SCIENCE, PURE AND APPLIED CHEMISTRY , A37(1 & 2), 37-47 CODEN: JSPCE6; ISSN: 1060-1325, 2000, XP009129181
- ZILKHA, ALBERT ET AL: "Syntheses of amide derivatives of DL-.beta.-carboxy-.gamma.-aminobutyric acid" JOURNAL OF ORGANIC CHEMISTRY , 28(8), 2007-9 CODEN: JOCEAH; ISSN: 0022-3263, 1963, XP002568809
- WATANABE, HIROYUKI ET AL: "Radical polymerization of N-substituted itaconamic esters and itaconamides" JOURNAL OF POLYMER SCIENCE, PART A: POLYMER CHEMISTRY , 32(11), 2085-91 CODEN: JPACEC; ISSN: 0887-624X, 1994, XP002568810
- WATANABE, HIROYUKI ET AL: "Polymerization of N-alkyl-substituted itaconimides and N-(alkyl-substituted phenyl)itaconimides and characterization of the resulting polymers" JOURNAL OF POLYMER SCIENCE, PART A: POLYMER CHEMISTRY , 32(11), 2073-83 CODEN: JPACEC; ISSN: 0887-624X, 1994, XP002568811

## Beschreibung

Die vorliegende Erfindung betrifft Mischungen enthaltend Itaconsäure oder Itaconsäurederivate und primäre Amine, und deren Verwendung als Ausgangsprodukt für die Herstellung von 1,3- und 1,4-Alkylmethylpyrrolidonen.
Weiterhin betrifft die vorliegende Erfindung ein Verfahren zur Herstellung von 1,3- und 1,4-Alkylmethylpyrrolidonen durch Umsetzung von erfindungsgemäßen Mischungen mit Wasserstoff in Gegenwart von Hydrierkatalysatoren. Weiterhin beschrieben sind Mischungen enthaltend 1,3- Alkylmethylpyrrolidon und 1,4-Alkylmethylpyrrolidon sowie die Verwendung dieser Mischungen.

N-Alkyl-pyrrolidone sind wichtige Produkte in der chemischen Industrie, die in einer Vielzahl von Anwendungen zum Einsatz kommen.
N-Alkyl-pyrrolidone sind thermisch stabile, chemisch weitgehend inerte, farblose, niedrigviskose und aprotische Lösungsmittel mit breiter Anwendbarkeit. So sind N-Methylpyrrolidon (NMP) und N-Ethyl-pyrrolidon (NEP) sowie die höheren Homologen als Lösungsmittel, Verdünnungsmittel, Extraktionsmittel, Reinigungsmittel, Entfettungsmittel, Adsorptionsmittel und/oder Dispergierungsmittel einsetzbar.
NMP findet Anwendung in der Extraktion von reinen Kohlenwasserstoffen in der petrochemischen Verarbeitung, in der Reinigung und Abtrennung von Gasen wie Acetylen 1,3-Butadien oder Isopren, in der Aromatenextraktion, beispielsweise im Distapex-Verfahren der LURGI GmbH, in der Sauergaswäsche und in der Schmierölextraktion. Außerdem kann NMP als Lösungsmittel für Polymer-Dispersionen, beispielsweise für Polyurethan-Dispersionen, verwendet werden.
NMP ist auch ein gutes Lösungsmittel für viele Kunststoffe wie Polyvinylchlorid (PVC), Polyurethane (PU), Acrylate oder Butadien-Acrylnitril-Copolymere und wird bei deren Verarbeitung eingesetzt.
NMP wird auch als Reinigungsmittel bei der Entfernung von Farb- und Lackresten eingesetzt sowie als Abbeizer und als Reinigungsmittel für Metall-, Keramik-, Glas- und Kunststoffoberflächen.
Ebenso ist NMP Lösungsmittel oder Cosolvens für die Formulierung von Wirkstoffen im Pflanzenschutz.
NEP und andere N-Alkyl-pyrrolidone können NMP in vielen Anwendungen ersetzen und zeigen darüber hinaus in vielen Fällen zusätzlich vorteilhafte Eigenschaften (WO-A-2005/090447, BASF SE).

Die Herstellung von N-Alkyl-pyrrolidonen ist bekannt.
N-Alkyl-pyrrolidone können beispielsweise durch Umsetzung von gamma-Butyrolacton (γ-BL) mit Monoalkylaminen unter Freisetzung von einem Äquivalent Wasser erhalten werden, z. B. analog Ullmann's Encyclopedia of Industrial Chemistry, Band A22, 5. Aufl., S. 459 (1993) oder analog DE-A-19 626 123 (BASF SE).
Ebenso können N-Alkyl-pyrrolidone aus Maleinsäureanhydrid oder anderen Dicarbonsäurederivaten und Monoethylaminen in Gegenwart von Wasserstoff und einem Hydrierkatalysator hergestellt werden, z. B. gemäß EP-A-745 598 (Bayer AG) oder WO-A-02/102773 (BASF SE).

Neben N-Alkyl-pyrrolidonen sind auch substituierte N-Alkyl-pyrrolidone bekannt.
Durch die Substitution können die N-Alkyl-pyrrolidone in ihren Anwendungs- und Verarbeitungseigenschaften modifiziert werden.

Ein Beispiel für eine solche Modifikation sind N-Alkyl-pyrrolidone mit einem oder mehreren Alkylsubstituenten, die an den Positionen 3 und 4 des Pyrrolidon-Ringes gebunden sind.
Alkyl-substituierte N-Alkyl-pyrrolidone können beispielsweise gemäß der Offenbarung von EP-A1-0027022 durch Umsetzung von Alkylaminen und Wasserstoff mit substituierten cyclischen Anhydriden/Imiden in Gegenwart von Ruthenium-Katalysatoren, hergestellt werden.
WO-A1-2005051907 offenbart die Umsetzung von Dicarbonsäuren oder deren Derivate mit Wasserstoff und Aminen in Gegenwart von Ruthenium- oder Osmium-Katalysatoren.
In der US 4,731,454 wird die Reduktion von cyclischen Imiden an Cobalt-Katalysatoren beschrieben, wobei die entsprechenden N-Alkyl-pyrrolidone erhalten werden.
Die Umsetzung von gesättigten cyclischen Carbonsäureimiden oder von gesättigten Ammoniumsalzen der Dicarbonsäuren in Gegenwart von auf Kohlenstoff geträgerten Edelmetallkatalysatoren wird in der WO 02/102772 offenbart.
Die DE-A1-1620191 beschreibt die Umsetzung von Alkylbernsteinsäure mit primären Aminen und Wasserstoff in Gegenwart eines Hydrierkatalysators zu den entsprechenden N-substituierten Derivaten von α-Pyrrolidonen, die an den Kohlenstoffatomen in Stellung 3 und/oder 4 alkyliert sind.

Die voranstehend genannten Literaturstellen offenbaren nur den Einsatz von gesättigten Dicarbonsäuren und Dicarbonsäurederivaten, sowie von Dicarbonsäuren bzw. Dicarbonsäurederivaten, die eine ungesättigte Hauptkette aufweisen, wie Maleinsäure und deren Derivate, als geeignete Ausgangsstoffe für die Herstellung von Pyrrolidonen. Es wird nicht beschrieben, dass Dicarbonsäuren oder Dicarbonsäurederivate, die eine ungesättigte Seitenkette oder sogar eine endständige Doppelbindung in der Seitenkette aufweisen, zu den entsprechenden Pyrrolidonen umgesetzt werden können.
Ein Beispiel für eine Dicarbonsäure mit einer ungesättigen, endständigen Seitenkette ist Itaconsäure.
Es ist bekannt, dass bei der Umsetzung von Itaconsäure mit primären Aminen in einer exothermen Reaktion die entsprechenden 4-Carboxypyrrolidone oder 4-Carbamido-pyrrolidone gebildet werden, welche durch Addition der primären Amine an die ungesättigte Seitenkette entstehen ( siehe Imamura et al., Chemical & Pharmaceutical Bulletin (2004), 52(1), 63-73, Paytash et al., Journal of the American Chemical Society (1950), 72, 1415-1416 und Southwick et al., Journal of Organic Chemistry (1956), 21, 1087-1095). Demgemäß ist die direkte Umsetzung von Itaconsäure mit primären Aminen und Wasserstoff zu den entsprechenden Pyrrolidonen nicht offenbart, da die Bildung der 4-Carboxypyrrolidone oder 4-Carbamidopyrrolidone einsetzt, bevor die Hydrierung zu den gewünschten Pyrrolidonen eintritt.
Auch stickstoffhaltige Derivate der Itaconsäure, wie deren Amide oder Imine, können unter den Bedingungen der Hydrierung unter Addition des Stickstoffs an die Doppelbindung zu unerwünschten cyclischen Nebenprodukten reagieren.
Itaconsäure und ihre Derivate unterscheiden sich durch diese Reaktivität wesentlich von ihren Strukturisomeren Citraconsäure und Mesaconsäure und deren Derivaten, die eine ungesättigte Hauptkette aufweisen und sich daher durch die in den voranstehenden Literaturstellen beschriebenen Methoden zu Alkylmethylpyrrolidonen umsetzen lassen.
Um Itaconsäure dennoch als Ausgangsstoff für die Herstellung von methylsubstituierten N-Alkylpyrrolidonen verwenden zu können, kann 2-Methylbernsteinsäure gemäß der Lehre von DE-A1-1620191 mit primären Aminen mit Wasserstoff in Gegenwart von Hydrierungskatalysatoren umgesetzt werden. 2-Methylbernsteinsäure kann wiederum durch Hydrierung von Itaconsäure hergestellt werden (CN-A1-1609089). Dies bedeutet jedoch, dass vor der Umsetzung von 2-Methylbernsteinsäure mit primären Aminen zu Pyrrolidonen, das Ausgangsprodukt 2-Methylbernsteinsäure in einer vorangeschalteten Reaktionsstufe erzeugt werden muss.

Die Aufgabe der vorliegenden Erfindung war die Bereitstellung eines Verfahrens zur Herstellung von 1,3- und 1,4-Alkylmethylpyrrolidonen, in dem Itaconsäure oder Itaconsäurederivate mit primären Aminen und Wasserstoff mit einer hohen Selektivität und Ausbeute umgesetzt werden können. Insbesondere sollte die Bildung von unerwünschten Nebenprodukten, insbesondere von 4-Carboxypyrrolidon oder 4-Carbamido-pyrrolidon, weitest gehend vermieden werden. Speziell bei der Herstellung von 1,3-Dimethylpyrrolidon und/oder 1,4-Dimethylpyrrolidon sollte die Bildung von toxischem N-Methylpyrrolidon vermieden werden. N-Methylpyrrolidon kann beispielsweise durch Folgereaktionen (Decarboxylierung/Hydrierung) aus 4-Carboxypyrrolidon und dessen Derivaten entstehen.
Eine weitere Aufgabe bestand darin, ein vereinfachtes Verfahren zur Herstellung von 1,3- und 1,4-Alkylmethylpyrrolidonen zur Verfügung zu stellen, das auf Itaconsäure basiert, das aber nicht die separate Hydrierung von Itaconsäure zu der entsprechenden 2-Methylbernsteinsäure erforderlich macht. Somit sollte ein Verfahren mit einer hohen Verfahrensökonomie bereitgestellt werden, das zudem technisch einfach zu realisieren ist. Ein weiteres Ziel dieser Erfindung war die Herstellung von 1,3- und 1,4-Alkylmethylpyrrolidonen unter Verwendung von Itaconsäure bzw. Itaconsäurederivaten, die auf Basis von nachwachsenden Rohstoffen hergestellt werden können. Das Zurückgreifen auf nachwachsende Rohstoffe kann zu einer Schonung von erschöpfbaren Ressourcen beitragen und ermöglicht ein nachhaltiges Wirtschaften.

Die Aufgabe wurde gelöst durch die Verwendung von Mischungen enthaltend Itaconsäure oder ein Itaconsäurederivat und ein primäres Amin der Formel (I),

R-NH₂ (I)

wobei man als Itaconsäurederivat einen Itaconsäure-Ester, ein Itaconsäure-Amid, ein Itaconsäure-Halogenid, ein Salz der Itaconsäure oder ein Itaconsäure-Imin einsetzt und das molare Verhältnis von primärem Amin zu Itaconsäure oder dem Itaconsäurederivat im Bereich von 0,5:1 bis 20:1 liegt, welche dadurch gekennzeichnet ist, dass die Mischung 50 Molprozent oder weniger 4-Carboxypyrrolidone der Formel (II), Derivate der 4-Carboxypyrrolidone der Formel (IX) und 4-Carbamidopyrrolidone der Formel (III) bezogen auf die eingesetzte Itaconsäure oder das eingesetzte Itaconsäurederivat enthält, 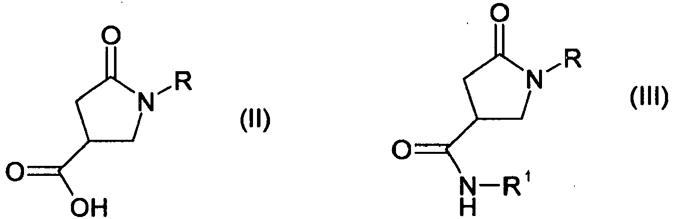 und in der R einen linearen oder verzweigten gesättigten aliphatischen Rest mit 1 bis 24 C-Atomen oder einen gesättigten cycloaliphatischen Rest mit 3 bis 24 C-Atomen bedeutet 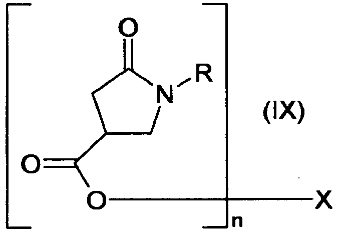 wobei n eine ganze Zahl von 1 bis 4
und X entweder H, R, ein Metall M oder NH₃R bedeutet, wobei R die voranstehend genannte Bedeutung hat,
zur Herstellung von 1,3- Alkylmethylpyrrolidonen der allgemeinen Formel (VI) und/oder 1,4-Alkylmethylpyrrolidonen allgemeinen Formel (VII) 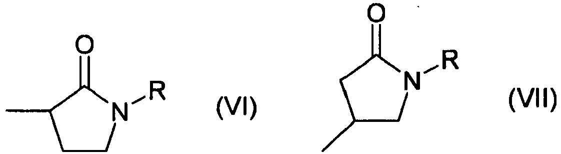 wobei R die oben genannte Bedeutung hat.

Die erfindungsgemäß verwendete Mischung enthält ein primäres Amin der allgemeinen Formel (I)

R-NH₂ (I)

in der R einen linearen oder verzweigten gesättigten aliphatischen Rest mit 1 bis 24 C-Atomen, bevorzugt C₁₋₁₂-Alkyl, wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, Cyclopentylmethyl, n-Heptyl, iso-Heptyl, Cyclohexylmethyl, n-Octyl, 2-Ethyl-hexyl, n-Nonyl, iso-Nonyl, n-Decyl, iso-Decyl, n-Undecyl, n-Dodecyl, iso-Dodecyl,
besonders bevorzugt C₁₋₈-Alkyl, wie Methyl, Ethyl, n-Propyl, iso-Propyl, n-Butyl, iso-Butyl, sec.-Butyl, tert.-Butyl, n-Pentyl, n-Hexyl, n-Octyl und 2-Ethyl-hexyl,
ganz besonders bevorzugt C₁₋₄-Alkyl, wie Methyl, Ethyl, n-Propyl, iso-Propyl, n-Butyl, iso-Butyl, sec.-Butyl, tert.-Butyl,
insbesondere bevorzugt Methyl,
oder einen
gesättigten cycloaliphatischen Rest mit 3 bis 24 C-Atomen, bevorzugt C₄₋₈-Cycloalkyl, wie Cyclobutyl, Cyclopentyl, Cyclohexyl, Cycloheptyl und Cyclooctyl, besonders bevorzugt Cyclopentyl und Cyclohexyl, bedeutet.

Bevorzugte primäre Amine sind Methylamin, Ethylamin, n-Propylamin, iso-Propylamin, n-Butylamin, iso-Butylamin, sec.-Butylamin, tert.-Butylamin, n-Pentylamin, iso-Pentylamin und 2-Ethyl-hexylamin.
Ganz besonders bevorzugte primäre Amine sind Methylamin, Ethylamin, n-Propylamin, iso-Propylamin, n-Butylamin, iso-Butylamin, sec.-Butylamin und tert.-Butylamin, insbesondere Methylamin.

Die erfindungsgemäße verwendeten Mischungen enthalten weiterhin Itaconsäure oder ein Itaconsäurederivat.

In einer besonders bevorzugten Ausführungsform enthält die erfindungsgemäß verwendete Mischung Itaconsäure. Itaconsäure wird bevorzugt auf Basis von nachwachsenden Rohstoffen hergestellt.
Itaconsäure kann beispielsweise durch Destillation von Zitronenschalen gewonnen werden.
In der Regel wird Itaconsäure kommerziell durch Fermentation von Kohlenhydraten aus ungereinigten Rohr- oder Rübenzucker oder aus Melasse hergestellt. Üblicherweise entsteht durch Verwendung der Enzyme Aspergillus terreus oder Aspergillus itaco-nicus (Jakubowska, in Smith u. Pateman (Hrsg.), Genetics and Physiology of Aspergillus, London: Academic Press 1977; Miall, in Rose (Hrsg.), Economic Microbiology, Vol. 2, S. 47 - 119, London: Academic Press 1978) die Itaconsäure aus Zitronensäure, die im Zitronsäurezyklus gebildet wird. Die Zitronensäure wird in der Regel zunächst durch die Aconitathydratase zur cis-Aconitsäure umgesetzt, die dann durch die Aconitatdecarboxylase zur Itaconsäure decaboxyliert wird (US 3,044,941).
Das Vorhandensein oder die Abwesenheit der strukturisomeren Säuren Citraconsäure und Mesaconsäure, welche aus Itaconsäure gewonnen werden können, in den erfindungsgemäßen Mischungen ist unkritisch.

Die erfindungsgemäß verwendete Mischung kann auch ein Itaconsäurederivat enthalten.

Das Itaconsäurederivat ist bevorzugt ein Itaconsäure-Ester.
Itaconsäure kann nach bekannten Methoden verestert werden.
Cowie et al. (J.M. G. Cowie et al., Polyner 18 (1977), 612-616) offenbaren die Säure katalysierte Veresterung von Itaconsäure, bei der ein Produktgemisch aus dem Di-Ester der Itaconsäure und dem entsprechenden Itaconsäure-4-alkylester erhalten wird. Der Itaconsäure-4-alkylester bildet sich in der Regel auch bei der Veresterung des Anhydrids als Hauptprodukt, da die 4-Position reaktiver ist, als die 2-Position. Bevorzugte Di-Ester der Itaconsäure sind Dimethyl-Itaconsäureester, Diethyl-Itaconsäureester, Di-n-propyl-Itaconsäureester, Di-n-butyl-Itaconsäureester, Di-n-pentyl-Itaconsäureester, Di-n-2-Methylpentyl-Itaconsäureester, Di-n-hexyl-Itaconsäureester und Di-2-Ethylhexyl-Itaconsäureester.
Bevorzugte Mono-Ester der Itaconsäure sind 4-Methyl-Itaconsäureester, 4-Ethyl-Itaconsäureester, 4-n-Propyl-Itaconsäureester und 4-n-Butyl-Itaconsäureester, 4-n-pentyl-Itaconsäureester, 4-(2-Methylpentyl)-Itaconsäureester, 4-n-hexyl-Itaconsäureester und 4-(2-Ethylhexyl)-Itaconsäureester.

Ein weiteres bevorzugtes Itaconsäurederivat ist Itaconsäure-Amid.
Die Synthese von Itaconsäure-Amiden wird in der Dissertation von Christine Rüdiger ("Synthesen und Untersuchungen zum Polymerisationsverhalten von Itaconsäurederivaten", Universität Wuppertal, Seite 38 (http://elpub.bib.uni-wuppertal.de/edocs/ dokumente/fb09/diss2000/ruediger; internal&action=buildframes.action)) beschrieben.
Zur Synthese der Itaconsäure-Amide geht man in der Regel von Itaconsäureanhydrid aus, das in THF mit dem entsprechenden primären Amin umgesetzt wird. Das Produkt kann durch Umkristallisation, beispielsweise aus Cyclohexan, isoliert werden.
In der Regel werden die Mono-Amide der Itaconsäure (Iltaconsäure-4-amide) erhalten, da wie voranstehend erläutert, die die 4-Position reaktiver ist, als die 2-Position.
Es können aber auch Di-Amide oder Mischungen von Mono- und Di-Amiden der Itaconsäure eingesetzt werden.

Auch Itaconsäure-Imine können als bevorzugte Itaconsäurederivate in die erfindungsgemäß verwendete Mischung eingesetzt werden.

Andere Itaconsäurederivate, die in erfindungsgemäß verwendeten Mischungen eingesetzt werden können, sind Itaconsäurederivate, die sich chemisch ähnlich zu einem oder mehreren der zuvor genannten Itaconsäurederivate verhalten, beispielsweise die entsprechenden Itaconsäure-Halogenide, wie Itaconsäure-Chlorid oder Itaconsäure-Bromid.
Als Itaconsäurederivate können aber auch Salze der Itaconsäure oder Mischungen von Salzen der Itaconsäure eingesetzt werden.
Salze der Itaconsäure sind beispielsweise Metallsalze der Itaconsäure, wie Alkalimetallsalze der Itaconsäure, Erdalkalimetallsalze der Itaconsäure, Aluminiumsalze der Itaconsäure oder Eisensalze der Itaconsäure.
Als Salze der Itaconsäure können aber auch Ammoniumsalze der Itaconsäure oder Alkylammoniumsalze der Itaconsäure eingesetzt werden.

Das molare Verhältnis von Amin zu Itaconsäure bzw. dem eingesetzten Itaconsäurederivat beträgt erfindungemäß 0,5:1 bis 20:1.

Werden Itaconsäure oder Mono-Ester der Itaconsäure oder Di-Ester der Itaconsäure eingesetzt, so beträgt das molare Verhältnis von Amin zu Itaconsäure bzw. Itaconsäure-Ester bevorzugt 1:1 bis 20:1, besonders bevorzugt 1,5:1 bis 10:1 und besonders bevorzugt 2:1 bis 8:1.
Wird als Itaconsäurederivat das Mono-Amid der Itaconsäure oder das Mono-Imin der Itaconsäure eingesetzt, so beträgt das molare Verhältnis von Amin zu dem genannten monosubstituierten Derivaten der Itaconsäure vorzugsweise 0,5:1 bis 20:1, besonders bevorzugt 1:1 bis 10:1 und ganz besonders bevorzugt 2:1 bis 8:1.
Wird als Itaconsäurederivat das Di-Amid der Itaconsäure oder das Di-Imin der Itaconsäure eingesetzt, so beträgt das molare Verhältnis von Amin zum den genannten disubstituierten Derivaten der Itaconsäure vorzugsweise 0,5:1 bis 20:1, besonders bevorzugt 0,5:1 bis 10:1 und ganz besonders bevorzugt 1:1 bis 8:1.

Die erfindungsgemäß verwendeten Mischungen enthalten einen Anteil an 4-Carboxypyrrolidonen der Formel (II), an Derivaten der 4-Carboxypyrrolidone der Formel (IX) und an 4-Carbamidopyrrolidonen der Formel (III), von weniger als 50 Molprozent bezogen auf die eingesetzte Itaconsäure oder das eingesetzte Itaconsäurederivat vorzugsweise weniger als 30 Molprozent, besonders bevorzugt weniger als 10 Molprozent, ganz besonders bevorzugt weniger als 5 Molprozent und insbesondere bevorzugt weniger als 1 Molprozent, jeweils bezogen auf die eingesetzte Itaconsäure oder das eingesetzte Itaconsäurederivat.
In der Regel liegen 4-Carboxypyrrolidone der Formel (II) in der erfindungsgemäße verwendeten Mischung nicht in Form der freien Säure vor, sondern als entsprechende Derivate der 4-Carboxypyrrolidone der Formel (IX), die sich in der erfindungsgemäßen Mischung bilden können.
Solche Derivate der 4-Carboxypyrrolidone der Formel (IX) können durch die allgemeine Formel (IX) dargestellt werden, 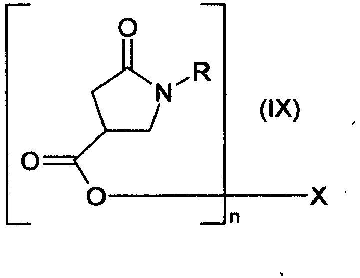 wobei n eine ganze Zahl von 1 bis 4
und X entweder H, R, ein Metall M oder NH₃R bedeutet, wobei R die voranstehend genannte Bedeutung hat.
Vorzugsweise bedeutet n eine ganze Zahl von 1 bis 2, besonders bevorzugt ist n gleich 1.
X kann ein organischer Rest R sein, wobei R die voranstehend genannte Bedeutung hat.
M ist vorzugsweise ein ein- bis vierwertiges Metall, bevorzugt ein Metall der Alkaligruppe oder Erdalkaligruppe, wie Ca oder Mg, besonders bevorzugt der Alkaligruppe, wie Li, Na oder K.
Besonders bevorzugt ist X gleich NH₃R, wobei R die voranstehend genannte Bedeutung hat.
Wenn X gleich M oder NH₃R bedeuten, liegt die Verbindung der allgemeinen Formel (VIII) bevorzugt in Salzform vor, wobei die negative Ladung bevorzugt an der Carboxylat-Gruppe (COO⁻) und die positive Ladung vorzugsweise an dem Substituenten X lokalisiert ist, beispielsweise als M⁺ oder N⁺RH₃.

Die im Stand der Technik bekannten Mischungen von Itaconsäure bzw. Itaconsäurederivaten und primären Aminen enthalten einen höheren Gehalt an 4-Carboxypyrrolidonen der Formel (II) oder 4-Carbamidopyrrolidone der Formel (III), da sich diese Verbindungen, wie voranstehend beschrieben, spontan durch Addition der primären Amine an die Doppelbindung der Itaconsäure bilden.

Es wurde nun gefunden, das die erfindungsgemäß verwendeten Mischungen von primären Amin und Itaconsäure bzw. Itaconsäurederivaten mit einem geringeren Anteil an 4-Carboxypyrrolidonen der Formel (II), Derivaten der 4-Carboxypyrrolidone der Formel (IX) und 4-Carbamidopyrrolidonen der Formel (III) erhältlich sind, in dem man das primare Amin mit der Itaconsäure bzw. dem Itaconsäurederivat in Kontakt bringt, wobei die Temperatur während des Inkontaktbringens 100°C oder weniger beträgt.
Demgemäß betrifft die vorliegende Erfindung ein
Verfahren zur Herstellung einer Mischung enthaltend Itaconsäure oder ein Itaconsäurederivat und ein primäres Amin der Formel (I),

R-NH₂ (I)

wobei das molare Verhältnis von primärem Amin zu Itaconsäure oder dem Itaconsäurederivat im Bereich von 0,5:1 bis 20:1 liegt, dadurch gekennzeichnet, dass die Mischung 50 Molprozent oder weniger 4-Carboxypyrrolidone der Formel (II), Derivate der 4-Carboxypyrrolidone der Formel (II) und 4-Carbamidopyrrolidone der Formel (III) bezogen auf die eingesetzte Itaconsäure oder das eingesetzte Itaconsäurederivat enthält, 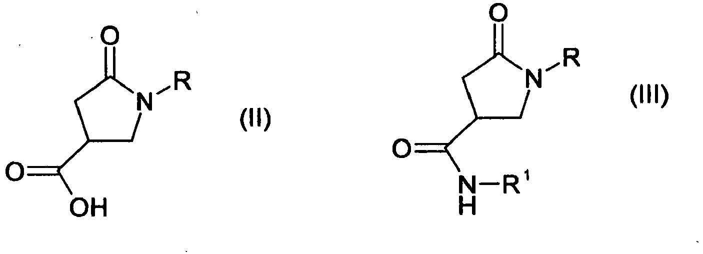 und in der R einen linearen oder verzweigten gesättigten aliphatischen Rest mit 1 bis 24 C-Atomen oder einen gesättigten cycloaliphatischen Rest mit 3 bis 24 C-Atomen bedeutet, dadurch gekennzeichnet, dass man das primäre Amin mit der Itaconsäure oder dem Itaconsäurederivat in Kontakt bringt, wobei die Temperatur während des Inkontaktbringens 100°C oder weniger beträgt.

Das Inkontakbringen der Itaconsäure bzw. dem Itaconsäurederivat mit dem primären Amin erfolgt bevorzugt durch Vermischen der beiden Komponenten.
Vorzugsweise sollte durch das Vermischen von Itaconsäure bzw. dem Itaconsäurederivat und dem primären Amin eine homogene Mischung erhalten werden. Die Homogenisierung kann beispielsweise durch intensives Rühren erfolgen, vorzugsweise mit einem Rührer, beispielsweise einem Planetenrührer, Ankerrührer, Balkenrührer, Propeller, Blattrührer, Dissolverscheiben oder Intermig-Rührer. Weitere geeignete Rührerkonfigurationen sind dem Fachmann bekannt.
Erfindungsgemäß beträgt die Temperatur während des Inkontraktbringens von Itaconsäure bzw. dem Itaconsäurederivat mit dem primären Amin 100°C und weniger, bevorzugt 75°C und weniger, ganz besonders bevorzugt 50°C und weniger, insbesondere bevorzugt 25°C und weniger. Vorzugweise liegt die Temperatur während des Inkontraktbringens von Itaconsäure bzw. dem Itaconsäurederivat mit dem primären Amin im Bereich von -10 bis 100°C, vorzugsweise im Bereich von -5 bis 75°C, besonders bevorzugt im Bereich von 0 bis 50°C und insbesondere bevorzugt im Bereich von 0 bis 25°C.
Üblicherweise wird das Inkontaktbringen von Itaconsäure bzw. dem Itaconsäurederivat und primären Amin in einem gekühlten Doppelmantelgefäß durchgeführt.
Das Inkontaktbringen von primärem Amin und Itaconsäure bzw. dem Itaconsäurederivat erfolgt bevorzugt in einem Lösungsmittel. Das Lösungsmittel sollte mit dem eingesetzten primären Amin eine ausreichende Mischbarkeit aufweisen.
Vorzugsweise sollte das Lösungsmittel auch eine gute Mischbarkeit mit der Mischung aus Itaconsäure bzw. dem Itaconsäurederivat und dem primären Amin aufweisen.
Ein solches Lösungsmittel lässt sich durch Löseversuche von primärem Amin und Lösungsmittel bei den entsprechenden Temperaturen, bei denen das primäre Amin mit der Itaconsäure bzw. dem Itaconsäurederivat in Kontakt gebracht wird, ermitteln. Bevorzugte Lösungsmittel sind Wasser, Methanol, Ethanol, n-/iso-Propanol, n-/iso-Butanol, THF, Dimethylformamid , Dimethylacetamid, Alkylmethylpyrrolidone oder N-Alkylpyrrolidone.
Besonders bevorzugt wird Wasser als Lösungsmittel eingesetzt.
Die Anteil an Itaconsäure bzw. dem Itaconsäurederivat im Lösungsmittel beträgt in der Regel 5 bis 95 Gew.-% bezogen auf das eingesetzte Lösungsmittel, bevorzugt 10 bis 70 Gew.-% und besonders bevorzugt 20 bis 60 Gew.-%, jeweils bezogen auf das eingesetzte Lösungsmittel.
In der Regel wird das primäre Amin vorgelegt und die Itaconsäure bzw. das Itaconsäurederivat zugegeben.

Wie voranstehend beschrieben kann das primäre Amin zusammen mit einem Lösungsmittel vorgelegt werden. Die Itaconsäure bzw. das Itaconsäurederivat kann ebenfalls in einem Lösungsmittel gelöst sein. Dieses Lösungsmittel ist vorzugsweise dasselbe, das für die Lösung des primären Amins verwendet wird. Es kann aber auch ein anderes Lösungsmittel sein, vorausgesetzt, dass beide Lösungsmittel miteinander mischbar sind. Die Itaconsäure bzw. das Itaconsäurederivat kann aber auch in fester Form zu dem primären Amin bzw. zu der Mischung aus primärem Amin und Lösungsmittel zugegeben werden.

In einer besonders bevorzugten Ausführungsform enthält die Mischung ein Salz der allgemeinen Formel [A²⁻] [B⁺][C⁺], wobei [A²⁻] ein Anion der Formel (IV), 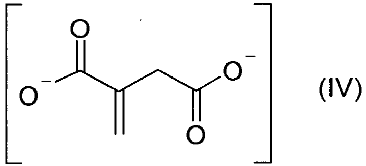 [B⁺] ein Kation der Formel (V) 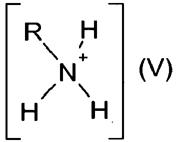 [C⁺] ein Kation der Formel (V) oder [H⁺] bedeutet und R die voranstehend genannte Bedeutung hat.
Das Ammoniumsalz der Itaconsäure mit der allgemeinen Formel [A²⁻] [B⁺][C⁺] kann in solvatisierter oder dissoziierter Form vorliegen, es kann aber auch in fester Salzform vorliegen.
Das Ammoniumsalz der Itaconsäure der allgemeinen Formel [A²⁻] [B⁺][C⁺] ist erhältlich durch Inkontaktbringen von Itaconsäure und einem primären Amin, wobei die Temperatur während des Inkontaktbringens 100°C oder weniger beträgt.
Das molare Verhältnis von primärem Amin zu Itaconsäure beträgt in der Regel 1:1 bis 2:1.
Beträgt das Verhältnis von primärem Amin zu Itaconsäure 1:1, so wird im Allgemeinen das Mono-Salz der Itaconsäure gebildet ([B⁺]=Kation der Formel (V) und [C⁺]=[H⁺]). Beträgt das Verhältnis von primärem, Amin zu Itaconsäure 2:1, so wird in der Regel das Di-Salz der Itaconsäure gebildet ([B⁺] und [C⁺]=Kation der Formel (V)).
Beträgt das molare Verhältnis von primärem Amin zu Itaconsäure zwischen 1:1 und 2:1, so werden in der Regel Mischungen des Mono- und des Disalzes erhalten.

Das molare Verhältnis von primärem Amin zu Itaconsäure kann auch mehr als 2:1 betragen. In der Regel wird dann eine Lösung bzw. Mischung des erfindungsgemäßen Ammoniumsalzes der Itaconsäure und überschüssigem primärem Amin erhalten. Bevorzugt beträgt das molare Verhältnis von Amin zu Itaconsäure 1:1 bis 20:1, besonders bevorzugt 1,5:1 bis 10:1 und besonders bevorzugt 2:1 bis 8:1.
Das Inkontaktbringen der Itaconsäure mit dem primären Amin erfolgt bevorzugt durch Vermischen der beiden Komponenten. Vorzugsweise sollte durch das Vermischen von Itaconsäure und dem primären Amin eine homogene Mischung erhalten werden. Die Homogenisierung kann beispielsweise durch intensives Rühren erfolgen, vorzugsweise mit einem Rührer, beispielsweise einem Planetenrührer, Ankerrührer, Balkenrührer, Propeller, Blattrührer, Dissolverscheiben oder Intermig-Rührer. Weitere geeignete Rührerkonfigurätionen sind dem Fachmann bekannt.
Erfindungsgemäß beträgt die Temperatur während des Inkontraktbringens von Itaconsäure mit dem primären Amin 100°C und weniger, bevorzugt 75°C und wenig, ganz besonders bevorzugt 50°C und weniger, insbesondere bevorzugt 25°C und weniger. Vorzugweise liegt die Temperatur während des Inkontaktbringens von Itaconsäure mit dem primären Amin im Bereich von -10 bis 100°C, vorzugsweise im Bereich von -5 bis 75°C, besonders bevorzugt im Bereich von 0 bis 50°C und insbesondere bevorzugt im Bereich von 0 bis 25°C.
Üblicherweise wird das Inkontaktbringen von Itaconsäure und primären Amin in einem gekühlten Doppelmantelgefäß durchgeführt.
In einer besonders bevorzugten Ausführungsform erfolgt das Inkontaktbringen von primärem Amin und Itaconsäure in einem Lösungsmittel. Das Lösungsmittel sollte mit dem eingesetzten primären Amin eine ausreichende Mischbarkeit aufweisen. Vorzugsweise sollte das Lösungsmittel auch eine gute Mischbarkeit mit der Mischung aus Itaconsäure und primärem Amin aufweisen. Ein solches Lösungsmittel lässt sich durch Löseversuche von primärem Amin und Lösungsmittel bei den entsprechenden Temperaturen, bei denen das primäre Amin mit der Itaconsäure in Kontakt gebracht wird, ermitteln.
Bevorzugte Lösungsmittel sind Wasser, Methanol, Ethanol, n-/iso-Propanol, n-/iso-Butanol, THF, Dimethylformamid, Dimethylacetamid, Alkylmethylpyrrolidone und N-Alkylpyrrolidone.
Besonders bevorzugt wird Wasser als Lösungsmittel eingesetzt.
Die Anteil an Itaconsäure im Lösungsmittel beträgt in der Regel 5 bis 95 Gew.-% bezogen auf das eingesetzte Lösungsmittel, bevorzugt 10 bis 70 Gew.-% und besonders bevorzugt 20 bis 60 Gew.-%, jeweils bezogen auf das eingesetzte Lösungsmittel.
In der Regel wird das primäre Amin vorgelegt und die Itaconsäure zugegeben.
Wie voranstehend beschrieben kann das primäre Amin zusammen mit einem Lösungsmittel vorgelegt werden. Die Itaconsäure kann ebenfalls in einem Lösungsmittel gelöst sein. Dieses Lösungsmittel ist vorzugsweise dasselbe, das für die Lösung des primären Amins verwendet wird. Es kann aber auch ein anderes Lösungsmittel sein, vorausgesetzt, dass beide Lösungsmittel miteinander mischbar sind. Die Itaconsäure kann aber auch in fester Form zu dem primären Amin bzw. zu der Mischung aus primärem Amin und Lösungsmittel zugegeben werden.
Die erfindungsgemäßen Ammoniumsalze der Itaconsäure liegen üblicherweise als Mischung mit dem Lösungsmittel oder überschüssigem primären Amin in solvatisierter bzw. dissoziierter Form vor.
Die erfindungsgemäßen Ammoniumsalze der Itaconsäure können isoliert werden, beispielsweise durch Ausfällung der Salze durch Zugabe einer Flüssigkeit, die eine geringe Löslichkeit für die Itaconsäuresalze aufweist.
Die Isolierung der Salze kann auch durch Verdampfen des Lösungsmittels erfolgen, wobei die Temperatur bei der Verdampfung nicht mehr als 100°C, vorzugsweise im Bereich von 0 bis 100°C, besonders bevorzugt im Bereich von 5 bis 75°C und ganz besonders bevorzugt im Bereich von 10 bis 50°C, betragen sollte, um die Bildung von Nebenprodukten bei der Verdampfung zu vermeiden.
In einer ganz besonders bevorzugten Ausführungsform wird jedoch die Mischung aus Lösungsmittel und Itaconsäuresalz, wobei das Itaconsäuresalz bevorzugt in dissoziierter Form vorliegt, ohne weitere Aufarbeitung direkt in die Hydrierung eingesetzt werden.

Die erfindungsgemäß verwendeten Mischungen enthaltend Itaconsäure oder ein Itaconsäurederivat und primäre Amine können auch in Gegenwart von Wasserstoff hergestellt werden. Erfolgt die Herstellung der erfindungsgemäß verwendeten Mischungen in Gegenwart von Wasserstoff, so sollte die Temperatur bei der Herstellung der Mischungen so gewählt werden, dass keine Bildung der unerwünschten Nebenprodukte auftritt. Vorzugsweise erfolgt deshalb die Herstellung der Mischung in Gegenwart von Wasserstoff bei 100°C und weniger, bevorzugt 75°C und weniger, ganz besonders bevorzugt bei 50°C und weniger, insbesondere bevorzugt bei 25°C und weniger. Vorzugweise liegt die Temperatur während des Inkontaktbringens von Itaconsäure bzw. dem Itaconsäurederivat mit dem primären Amin in Gegenwart von Wasserstoff im Bereich von -10 bis 100°C, vorzugsweise im Bereich von -5 bis 75°C, besonders bevorzugt im Bereich von 0 bis 50°C und insbesondere bevorzugt im Bereich von 0 bis 25°C.

Vor ihrer Weiterverarbeitung können die erfindungsgemäß verwendeten Mischungen gelagert werden. Die Lagerung erfolgt bevorzugt unter Bedingungen, bei denen eine Umsetzung der Mischung zu den unerwünschten Nebenprodukten 4-Carboxypyrrolidon, Derivaten von 4-Carboxypyrrolidon oder 4-Carbamidopyrrolidon vermieden wird. Vorzugsweise erfolgt die Lagerung bei Temperaturen von -10 bis 100°C, besonders bevorzugt bei Temperaturen von -5 bis 75°C und besonders bevorzugt bei Temperaturen von 0 bis 50°C, insbesondere bei Umgebungstemperatur, um die Bildung der Nebenprodukte 4-Carboxypyrrolidone der Formel (II), Derivate der 4-Carboxypyrrolidone der Formel (IX) oder 4-Carbamidopyrrolidone der Formel (III) zu verringern.

Ein Vorteil der vorliegenden Erfindung besteht darin, dass stabile Mischungen aus Itaconsäure bzw. Itaconsäurederivaten und primären Aminen bereitgestellt werden konnten, bei denen die Bildung von unerwünschten Nebenprodukten, wie 4-Carboxy-pyrrolidon, Derivate von 4-Carboxypyrrolidon der Formel (IX) oder 4-Carbamido-pyrrolidon weitestgehend vermieden wird. Diese Mischungen sind bei Umgebungstemperatur stabil und können somit ohne erhöhten technischen Aufwand gelagert und transportiert werden. Die erfindungsgemäß verwendeten Mischungen, die nur einen geringen Anteil von 4-Carboxypyrrolidon oder 4-Carbamidopyrrolidon enthalten, stellen somit ein geeignetes neues Ausgangsprodukt für die Herstellung von 1,3- und 1,4-Alkylmethylpyrrolidonen dar. Ausgehend von diesem Ausgangsprodukt können die entsprechenden 1,3- und 1,4-Alkylmethylpyrrolidone in hoher Selektivität und Ausbeute, bezogen auf die eingesetzte Itaconsäure bzw. Itaconsäurederivate erhalten werden. Weiterhin betrifft die vorliegende Erfindung ein Verfahren zur Herstellung von 1,3- Alkylmethylpyrrolidonen der allgemeinen Formel (VI) und/oder 1,4-Alkylmethylpyrrolidonen der allgemeinen Formel (VII) 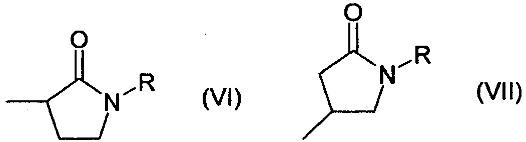 durch Umsetzung einer Mischung enthaltend Itaconsäure oder ein Itaconsäurederivat und ein primäres Amin der Formel (I) mit Wasserstoff in Gegenwart eines Hydrierkatalysators,

R-NH₂ (I)

wobei man als Itaconsäurederivat einen Itaconsäure-Ester, ein Itaconsäure-Amid, ein Itaconsäure-Halogenid, ein Salz der Itaconsäure oder ein Itaconsäure-Imin einsetzt und das molare Verhältnis von primärem Amin zu Itaconsäure oder dem Itaconsäurederivat im Bereich von 0,5:1 bis 20:1 liegt und die Mischung 50 Molprozent oder weniger 4-Carboxypyrrolidone der Formel (II), Derivate der 4-Carboxypyrrolidone der Formel (IX) und 4-Carbamidopyrrolidone der Formel (III) bezogen auf Itaconsäure oder Itaconsäurederivat enthält, und 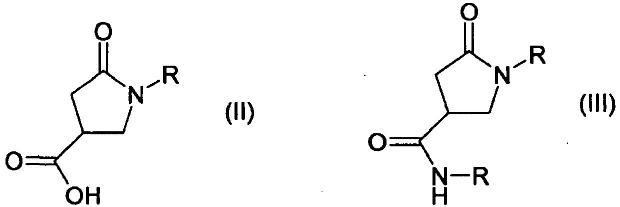 in der R die oben genannte Bedeutung hat, und Derivate der 4-carboxypyrrolidone der Formel (IX) wie oben beschrieben definiert sind.

Das erfindungsgemäße Verfahren findet in Gegenwart eines Hydrierkatalysators statt. Als Hydrierkatalysatoren können prinzipiell alle Hydrierkatalysatoren eingesetzt werden, die Nickel, Kobalt, Eisen, Kupfer, Ruthenium, Chrom, Mangan, Kupfer, Molybdän, Wolfram, Rhenium und/oder andere der Metalle der Gruppen 8 und/oder 9 und/oder 10 und/oder 11 des Periodensystems der Elemente (Periodensystem in der IUPAC-Fassung vom 22.06.2007) enthalten. Bevorzugt verwendet man Hydrierkatalysatoren, die Ruthenium, Rhodium, Kobalt und/oder Nickel enthalten. Besonders bevorzugt sind Katalysatoren die Rhodium, Ruthenium und/oder Kobalt enthalten. Ganz besonders bevorzugt sind Katalysatoren, die Rhodium enthalten.

Die oben genannten Hydrierkatalysatoren können in üblicher Weise mit Promotoren, beispielsweise mit Chrom, Eisen, Kobalt, Mangan, Thallium, Molybdän, Titan und/oder Phosphor dotiert werden.
Die katalytisch aktiven Metalle können als Vollkontakte oder auf Trägern eingesetzt werden. Als solche Träger kommen z.B. Kohlenstoff, wie Graphit, Ruß und/oder Aktivkohle, Aluminiumoxid (gamma, delta, theta, alpha, kappa, chi oder Mischungen daraus), Siliziumdioxid, Zirkoniumdioxid, Zeolithe, Alumosilicate oder deren Gemische in Betracht.

Die katalytisch aktiven Metalle können beispielsweise in Form von Schwammkatalysatoren, sogenannten Raney-Katalysatoren, eingesetzt werden. Als Raney-Katalysatoren werden bevorzugt Raney-Kobalt-Katalysatoren, Raney-Nickel-Katalysatoren und/oder Raney-Kupfer-Katalysatoren eingesetzt.
Die Herstellung von Hydrierkatalysatoren nach Raney erfolgt beispielsweise durch Behandlung einer Aluminium-Metall-Legierung mit konzentrierter Natronlauge, wobei das Aluminium ausgelaugt wird und ein metallischer Schwamm entsteht. Die Herstellung von Hydrierkatalysatoren nach Raney ist beispielsweise im Handbook of Heterogeneous Catalysis beschrieben (M. S. Wainright in G. Ertl, H. Knözinger, J. Weitkamp (eds.), Handbook of Heterogeneous Catalysis, Vol. 1, Wiley-VCH, Weinheim, Germany 1997, Seite 64 ff.). Solche Katalysatoren sind beispielsweise als Raney®-Katalysatoren von der Fa. Grace oder als Sponge Metal®-Katalysatoren der Johnson Matthey erhältlich.

Die in das erfindungsgemäße Verfahren einsetzbaren Hydrierkatalysatoren können auch durch Reduktion von sogenannten Katalysatorvorläufern hergestellt werden.
Der Katalysatorvorläufer enthält eine aktive Masse, die eine oder mehrere katalytisch aktive Komponenten und optional ein Trägermaterial enthält.
Bei den katalytisch aktiven Komponenten handelt es sich um sauerstoffhaltige Verbindungen der Metalle der oben genannten aktiven Metalle, beispielsweise um deren Metalloxide bzw. Hydroxide. (ggf. Beispiele), wie CoO, NiO, Mn₃O₄, CuO, RuO(OH)ₓ und/oder deren Mischoxide.
Im Rahmen dieser Anmeldung wird der Begriff katalytisch aktive Komponenten für oben genannte sauerstoffhaltige Metallverbindungen verwendet, soll aber nicht implizieren, dass diese sauerstoffhaltigen Verbindungen an sich bereits katalytisch aktiv sind. Die katalytisch aktiven Komponenten weisen in der Regel erst nach erfolgter Reduktion eine katalytische Aktivität in der erfindungsgemäßen Umsetzung auf.
Die Katalysatorvorläufer können nach bekannten Verfahren, z.B. durch Fällung, Auffällung oder Imprägnierung hergestellt werden.

In einer bevorzugten Ausführungsform werden Katalysatorvorläufer in das erfindungsgemäße Verfahren eingesetzt, die durch Tränkung (Imprägnierung) von Trägermaterialien hergestellt werden (getränkte Katalysatorvorläufer).

Die Trägermaterialien, die bei der Imprägnierung verwendet werden, können beispielsweise in Form von Pulvern oder Formkörpern, wie Strängen, Tabletten, Kugeln oder Ringen, eingesetzt werden. Für Wirbelbettreaktoren geeignetes Trägermaterial wird vorzugsweise durch Sprühtrocknung erhalten.
Als Trägermaterialien kommen beispielsweise Kohlenstoff, wie Graphit, Ruß und/oder Aktivkohle, Aluminiumoxid (gamma, delta, theta, alpha, kappa, chi oder Mischungen daraus), Siliziumdioxid, Zirkoniumdioxid, Zeolithe, Alumosilicate oder deren Gemische in Betracht.
Die Tränkung der obengenannten Trägermaterialien kann nach den üblichen Verfahren erfolgen (A. B. Stiles, Catalyst Manufacture - Laboratory and Commercial Preperations, Marcel Dekker, New York, 1983), beispielsweise durch Aufbringung einer Metallsalzlösung in einer oder mehreren Tränkstufen. Als Metallsalze kommen in der Regel wasserlösliche Metallsalze, wie die Nitrate, Acetate oder Chloride der entsprechenden katalytisch aktiven Komponenten oder Dotierelemente in Betracht, wie Co-Nitrat oder Co-Chlorid. Im Anschluss wird das getränkte Trägermaterial in der Regel getrocknet und ggf. calciniert.
Die Tränkung kann auch nach der sogenannten "incipient wetness-Methode" erfolgen, bei der das Trägermaterial entsprechend seiner Wasseraufnahmekapazität maximal bis zur Sättigung mit der Tränklösung befeuchtet wird. Die Tränkung kann aber auch in überstehender Lösung erfolgen.
Bei mehrstufigen Tränkverfahren ist es zweckmäßig, zwischen einzelnen Tränkschritten zu trocknen und ggf. zu calcinieren. Die mehrstufige Tränkung ist vorteilhaft dann anzuwenden, wenn das Trägermaterial in größerer Menge mit Metallsalzen beaufschlagt werden soll.
Zur Aufbringung mehrerer Metallkomponenten auf das Trägermaterial, kann die Tränkung gleichzeitig mit allen Metallsalzen oder in beliebiger Reihenfolge der einzelnen Metallsalze nacheinander erfolgen.

In einer weiteren bevorzugten Ausführungsform werden Katalysatorvorläufer über eine gemeinsame Fällung (Mischfällung) aller ihrer Komponenten hergestellt. Dazu wird in der Regel eine lösliche Verbindung der entsprechenden aktiven Komponente, der Dotierelemente und ggf. eine lösliche Verbindung eines Trägermaterials in einer Flüssigkeit in der Wärme und unter Rühren so lange mit einem Fällungsmittel versetzt, bis die Fällung vollständig ist.
Als Flüssigkeit wird in der Regel Wasser eingesetzt.
Als lösliche Verbindung der aktiven Komponenten kommen üblicherweise die entsprechenden Metallsalze, wie die Nitrate, Sulfate, Acetate oder Chloride, der Metalle der Gruppen 8 und/oder 9 und/oder 10 und/oder 11 des Periodensystems der Elemente (Periodensystem in der IUPAC-Fassung vom 22.06.2007) in Betracht.
Als wasserlösliche Verbindungen eines Trägermaterials werden in der Regel wasserlösliche Verbindungen von Ti, Al, Zr, Si etc., beispielsweise die wasserlöslichen Nitrate, Sulfate, Acetate oder Chloride dieser Elemente, verwendet.

Als wasserlösliche Verbindungen der Dotierelemente werden in der Regel wasserlösliche Verbindungen der Dotierelemente, beispielsweise die wasserlöslichen Nitrate, Sulfate, Acetate oder Chloride dieser Elemente, eingesetzt.

Katalysatorvorläufer können weiterhin durch Auffällung hergestellt werden.
Unter Auffällung wird eine Herstellmethode verstanden, bei der ein schwer lösliches oder unlösliches Trägermaterial in einer Flüssigkeit suspendiert wird und nachfolgend lösliche Verbindungen, wie lösliche Metallsalze, der entsprechenden Metalloxide zugegeben werden, welche dann durch Zugabe eines Fällungsmittels auf den suspendierten Träger aufgefällt werden (z.B. beschrieben in EP-A2-1 106 600, Seite 4, und A. B. Stiles, Catalyst Manufacture, Marcel Dekker, Inc., 1983, Seite 15).
Als schwer- bzw. unlösliche Trägermaterialien kommen beispielsweise Kohlenstoffverbindungen, wie Graphit, Ruß und/oder Aktivkohle, Aluminiumoxid (gamma, delta, theta, alpha, kappa, chi oder Mischungen daraus), Siliziumdioxid, Zirkoniumdioxid, Zeolithe, Alumosilicate oder deren Gemische in Betracht.
Das Trägermaterial liegt in der Regel als Pulver oder Splitt vor.
Als Flüssigkeit, in der das Trägermaterial suspendiert wird, wird üblicherweise Wasser eingesetzt.
Als lösliche Verbindungen kommen die voranstehend genannten löslichen Verbindungen der aktiven Komponenten bzw. der Dotierelemente in Betracht.

Bei den Fällungsreaktionen ist im Allgemeinen die Art der verwendeten löslichen Metallsalze nicht kritisch. Da es bei dieser Vorgehensweise vornehmlich auf die Wasserlöslichkeit der Salze ankommt, ist ein Kriterium ihre zur Herstellung dieser verhältnismäßig stark konzentrierten Salzlösungen erforderliche, gute Wasserlöslichkeit. Es wird als selbstverständlich erachtet, dass bei der Auswahl der Salze der einzelnen Komponenten natürlich nur Salze mit solchen Anionen gewählt werden, die nicht zur Störungen führen, sei es, indem sie unerwünschte Fällungsreaktionen verursachen oder indem sie durch Komplexbildung die Fällung erschweren oder verhindern.

Üblicherweise werden bei den Fällungsreaktionen die löslichen Verbindungen durch Zugabe eines Fällungsmittels als schwer- oder unlösliche, basische Salze gefällt.
Als Fällungsmittel werden bevorzugt Laugen, insbesondere Mineralbasen, wie Alkalimetallbasen eingesetzt. Beispiele für Fällungsmittel sind Natriumcarbonat, Natriumhydroxid, Kaliumcarbonat oder Kaliumhydroxid.
Als Fällungsmittel können auch Ammoniumsalze, beispielsweise Ammoniumhalogenide, Ammoniumcarbonat, Ammoniumhydroxid oder Ammoniumcarboxylate eingesetzt werden.

Die Fällungsreaktionen können z.B. bei Temperaturen von 20 bis 100 °C, besonders 30 bis 90 °C, insbesondere bei 50 bis 70 °C, durchgeführt werden.

Die bei den Fällungsreaktionen erhaltenen Niederschläge sind im Allgemeinen chemisch uneinheitlich und enthalten in der Regel Mischungen der Oxide, Oxidhydrate, Hydroxide, Carbonate und/oder Hydrogencarbonate der eingesetzten Metalle. Es kann sich für die Filtrierbarkeit der Niederschläge als günstig erweisen, wenn sie gealtert werden, d.h. wenn man sie noch einige Zeit nach der Fällung, gegebenenfalls in Wärme oder unter Durchleiten von Luft, sich selbst überlässt.

Die nach diesen Fällungsverfahren erhaltenen Niederschläge werden üblicherweise verarbeitet, indem sie gewaschen, getrocknet, calciniert und konditioniert werden.

Nach dem Waschen werden die Niederschläge im Allgemeinen bei 80 bis 200°C, vorzugsweise 100 bis 150°C, getrocknet und anschließend calciniert.

Die Calcinierung wird im Allgemeinen bei Temperaturen zwischen 300 und 800°C, vorzugsweise 400 bis 600°C, insbesondere bei 450 bis 550°C ausgeführt.

Nach der Calcinierung werden die durch Fällungsreaktionen erhaltenen Katalysatorvorläufer üblicherweise konditioniert.
Die Konditionierung kann beispielsweise dadurch erfolgen, dass man den Fällungskatalysator durch Vermahlen auf eine bestimmte Korngröße einstellt.
Nach der Vermahlung kann der durch Fällungsreaktionen erhaltenen Katalysatorvorläufer mit Formhilfsmitteln wie Graphit, oder Stearinsäure vermischt werden und zu Formkörpern weiterverarbeitet werden.
Gängige Verfahren der Formgebung sind beispielsweise im Ullmann [Ullmann's Encyclopedia Electronic Release 2000, Kapitel: "Catalysis and Catalysts", Seiten 28-32] und von Ertl et al. [Ertl, Knözinger, Weitkamp, Handbook of Heterogenoeous Catalysis, VCH Weinheim, 1997, Seiten 98 ff] beschrieben.
Wie in den genannten Literaturstellen beschrieben, können durch den Prozess der Formgebung Formkörper in jeglicher Raumform, z.B. rund, eckig, länglich oder dergleichen, z.B. in Form von Strängen, Tabletten, Granulat, Kugeln, Zylindern oder Körnern erhalten werden. Gängige Verfahren der Formgebung sind beispielsweise Extrusion, Tablettieren, d.h. mechanisches Verpressen oder Pelletieren, d.h. Kompaktieren durch kreisförmige und/oder rotierende Bewegungen.
Nach der Konditionierung bzw. Formgebung erfolgt in der Regel eine Temperung. Die Temperaturen bei der Temperung entsprechen üblicherweise den Temperaturen bei der Calcinierung.

Die durch Fällungsreaktionen erhaltenen Katalysatorvorläufer enthalten die katalytisch aktiven Komponenten in Form eines Gemisches ihrer sauerstoffhaltigen Verbindungen, d.h. insbesondere als Oxide, Mischoxide und/oder Hydroxide. Die so hergestellten Katalysatorvorläufer können als solche gelagert werden.

Vor ihrem Einsatz als Hydrierkatalysatoren werden Katalysatorvorläufer, die wie voranstehend beschrieben durch Imprägnierung oder Fällung erhalten wurden, werden in der Regel nach der Calcinierung bzw. Konditionierung durch Behandlung mit Wasserstoff vorreduziert.

Zur Vorreduktion werden die Katalysatorvorläufer im allgemeinen zunächst bei 150 bis 200°C über einen Zeitraum von 12 bis 20 Stunden einer Stickstoff-WasserstoffAtmosphäre ausgesetzt und anschließend noch bis zu ca. 24 Stunden bei 200 bis 400°C in einer Wasserstoffatmosphäre behandelt. Bei dieser Vorreduktion wird ein Teil der in den Katalysatorvorläufern vorliegenden sauerstoffhaltigen Metallverbindungen zu den entsprechenden Metallen reduziert, so dass diese gemeinsam mit den verschiedenartigen Sauerstoffverbindungen in der aktiven Form des Hydrierkatalysators vorliegen.
In einer bevorzugten Ausführungsform wird die Vorreduktion des Katalysatorvorläufers in demselben Reaktor vorgenommen, in dem anschließend die Hydrierung durchgeführt wird.

Der so gebildete Hydrierkatalysator kann nach der Vorreduktion unter einem Inertgas wie Stickstoff gehandhabt und gelagert werden oder unter einer inerten Flüssigkeit, zum Beispiel einem Alkohol, Wasser oder dem Produkt der jeweiligen Reaktion, für die der Hydrierkatalysator eingesetzt wird. Der Hydrierkatalysator kann nach der Vorreduktion aber auch mit einem Sauerstoff enthaltenden Gasstrom wie Luft oder einem Gemisch von Luft mit Stickstoff passiviert, d. h. mit einer schützenden Oxidschicht versehen werden.

Die Lagerung der Hydrierkatalysatoren, die durch Vorreduktion von Katalysatorvorläufern erhalten wurden, unter inerten Substanzen oder die Passivierung des Hydrierkatalysators ermöglichen eine unkomplizierte und ungefährliche Handhabung und Lagerung des Katalysators. Gegebenenfalls muss der Hydrierkatalysator vor Beginn der eigentlichen Reaktion dann von der inerten Flüssigkeit befreit werden bzw. die Passivierungsschicht z. B. durch Behandlung mit Wasserstoff oder einem Wasserstoff enthaltenden Gas aufgehoben werden.

Der Hydrierkatalysator kann vor seinem Einsatz in die Hydrierung von der inerten Flüssigkeit bzw. Passivierungsschicht befreit werden. Dies geschieht beispielsweise durch die Behandlung des Hydrierkatalysators mit Wasserstoff oder einem Wasserstoff enthaltendem Gas. Bevorzugt wird die Hydrierung direkt nach der Behandlung des Hydrierkatalysators im selben Reaktor vorgenommen, in dem auch die Behandlung des Hydrierkatalysators mit Wasserstoff oder einem Wasserstoff enthaltendem Gas erfolgte.

Katalysatorvorläufer können jedoch auch ohne Vorreduktion in das Verfahren eingesetzt werden, wobei sie dann unter den Bedingungen der Hydrierung durch den im Reaktor vorhandenen Wasserstoff reduziert werden, wobei sich der Hydrierkatalysator in der Regel in situ bildet.

In das erfindungsgemäße Verfahren wird Wasserstoff eingesetzt.
Der Wasserstoff kommt im Allgemeinen technisch rein zum Einsatz. Der Wasserstoff kann auch in Form eines Wasserstoff enthaltenden Gases, d.h. in Beimengungen mit anderen Inertgasen, wie Stickstoff, Helium, Neon, Argon oder Kohlendioxid zum Einsatz kommen. Als Wasserstoff enthaltende Gase können beispielsweise Reformerabgase, Raffineriegase usw. verwendet werden, wenn und soweit diese Gase keine Kontaktgifte für die eingesetzten Hydrierkatalysatoren, wie zum Beispiel CO enthalten. Bevorzugt wird jedoch reiner Wasserstoff bzw. im Wesentlichen reiner Wasserstoff in das Verfahren eingesetzt, beispielsweise Wasserstoff mit einem Gehalt von mehr als 99 Gew.-% Wasserstoff, bevorzugt mehr als 99,9 Gew.-% Wasserstoff, besonders bevorzugt mehr als 99,99 Gew.-% Wasserstoff, insbesondere mehr als 99,999 Gew.-% Wasserstoff.

In das erfindungsgemäße Verfahren werden weiterhin die voranstehend beschriebenen Mischungen enthaltend Itaconsäure oder ein Itaconsäurederivat und ein primäres Amin der Formel (I),

R-NH₂ (I)

wobei das molare Verhältnis von primärem Amin zu Itaconsäure oder dem Itaconsäurederivat im Bereich von 0,5:1 bis 20:1 liegt, dadurch gekennzeichnet, dass die Mischung 50 Molprozent oder weniger 4-Carboxypyrrolidone der Formel (II), Derivate der 4-Carboxypyrrolidone der Formel (II) und 4-Carbamidopyrrolidone der Formel (III) bezogen auf die eingesetzte Itaconsäure oder das eingesetzte Itaconsäurederivat enthält, 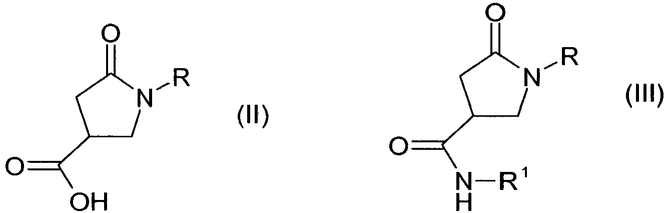 und in der R die voranstehend genannte Bedeutung hat, eingesetzt.

Vorzugsweise enthalten die Mischungen bereits ein Lösungsmittel. In der Regel wird der Mischung kein weiteres Lösungsmittel zugegeben, wenn die Mischung bereits Lösungsmittel enthält und der Anteil an Itaconsäure bzw. dem Itaconsäurederivat im Lösungsmittel von 5 bis 95 Gew.-% bezogen auf das eingesetzte Lösungsmittel, bevorzugt von 10 bis 70 Gew.-% und besonders bevorzugt von 20 bis 60 Gew.-%, jeweils bezogen auf das eingesetzte Lösungsmittel, beträgt. Beträgt der Anteil an Itaconsäure bzw. Itaconsäurederivat mehr als 95 Gew.-% bezogen auf das eingesetzte Lösungsmittel, so wird der Mischung vorzugsweise ein Lösungsmittel zugegeben. Vorzugsweise wird dasselbe Lösungsmittel zugegeben, das bereits zur Herstellung der erfindungsgemäßen Gemische eingesetzt wurde. Bevorzugte Lösungsmittel sind Wasser, Methanol, Ethanol, n-/iso-Propanol, n-/iso-Butanol, THF, Dimethylformamid, Dimethylacetamid, Alkylmethylpyrrolidone oder N-Alkylpyrrolidone, insbesondere Wasser.

Die Hydrierung wird üblicherweise bei einem Reaktionsdruck von 50 bis 300 bar, bevorzugt von 80 bis 280 bar, besonders bevorzugt von 100 bis 270 bar und ganz besonders bevorzugt bei einem Druck von 120 bis 250 bar durchgeführt. Die Druckhaltung bzw. Drucksteuerung erfolgt in der Regel über die Dosierung des Wasserstoffs.

Die Hydrierung erfolgt im Allgemeinen bei Reaktionstemperaturen von 100 bis 300°C, bevorzugt 120 bis 280°C, besonders bevorzugt 150 bis 250°C und ganz besonders bevorzugt 180 bis 220°C.

Die erfindungsgemäße Hydrierung kann kontinuierlich, diskontinuierlich oder semikontinuierlich durchgeführt werden.
Geeignete Reaktoren sind somit sowohl Rührkessel-Reaktoren als auch RohrReaktoren.
Typische Reaktoren sind beispielsweise Hochdruck-Rührkessel-Reaktoren, Autoklaven, Festbettreaktoren, Wirbelschichtreaktoren, Wariderbetten, zirkulierende Wirbelschichten, Salzbadreaktoren, Plattenwärmetauscher als Reaktoren, Hordenreaktoren mit mehreren Horden mit/oder ohne Wärmetausch bzw. Abzug/Zufuhr von Teilströmen zwischen den Horden, in möglichen Ausführungen als Radialstrom- oder Axialstromreaktoren, kontinuierlich gerührte Kessel, Blasenreaktoren usw., wobei jeweils der für die gewünschten Reaktionsbedingungen (wie Temperatur, Druck und Verweilzeit) geeignete Reaktor eingesetzt wird.
Bevorzugt wird die erfindungsgemäße Hydrierung in einem Hochdruck-Rührkessel-Reaktor, Festbettreaktor oder Wirbelschichtreaktor durchgeführt.

Bei der diskontinuierlichen Durchführung der Hydrierung erfolgt die Umsetzung bevorzugt in einem Hochdruck-Rührkessel-Reaktor. In einer besonders bevorzugten Ausführungsform erfolgt die Hydrierung in demselben Reaktor, in dem die erfindungsgemäβen Mischungen hergestellt wurden. Alternativ können die erfindungsgemäßen Mischungen in einem separaten Reaktor hergestellt werden und in den Reaktor, in dem die Hydrierung durchgeführt wird, überführt werden. Üblicherweise werden die erfindungsgemäßen Mischungen unter Wasserstoff, bei einem Druck von 1 bis 100 bar, vorzugsweise 5 bis 80 bar, zügig auf die Reaktionstemperatur aufgeheizt. In der Regel wird nach Erreichen der Reaktionstemperatur der Druck auf den Reaktionsdruck erhöht.
Bei der kontinuierlichen Durchführung der Hydrierung erfolgt die Umsetzung bevorzugt in einem Festbettreaktor, wobei die erfindungsgemäße Mischung bevorzugt in flüssiger Form der Reaktionszone zugeführt wird. Vorzugsweise wird die erfindungsgemäße Mischung erst unmittelbar vor oder während ihrer Zuführung in die Reaktionszone auf die Reaktionstemperatur erhitzt.

Die Verweilzeit beträgt in der Hydrierung -- bei der Durchführung in einem diskontinuierlichen Verfahren -- im Allgemeinen 15 Minuten bis 96 Stunden, bevorzugt 60 Minuten bis 72 Stunden, besonders bevorzugt 2 Stunden bis 48 Stunden.
Bei Durchführung in einem kontinuierlichen Verfahren beträgt die Verweilzeit im Allgemeinen 0,1 Sekunden bis 24 Stunden, bevorzugt 1 Minute bis 10 Stunden. Für die kontinuierlichen Verfahren bedeutet "Verweilzeit" in diesem Zusammenhang die Verweilzeit am Katalysator, für Festbettkatalysator somit die Verweilzeit im Katalysatorbett, für Wirbelschichtreaktoren wird der Syntheseteil des Reaktors (Teil des Reaktors, wo der Katalysator lokalisiert ist) betrachtet.

In einer bevorzugten Ausführungsform des Verfahrens wird die Verweilzeit so gewählt, dass bei der Hydrierung ein Umsatz von 99% und mehr, vorzugsweise 99,5% und mehr, besonders bevorzugt 99,8% und mehr und insbesondere bevorzugt 99,9% und mehr, jeweils bezogen auf die eingesetzte Itaconsäure bzw. das eingesetzte Itaconsäurederivat, erzielt wird. Diese bevorzugte Ausführungsform hat den Vorteil, dass in der Reaktionsmischung die Nebenkomponente 1,3-Alkylmethylsuccinimid zu 1,3- und 1,4-Alkylmethylpyrrolidon und ggf. zu 1,3-Alkylmethylpyrrolidin durchhydriert wird. Die Bildung der Nebenkomponente 1,3-Alkylmethylpyrrolidin zu Lasten der Nebenkomponente 1,3-Alkylmethylsuccinimid hat den Vorteil, dass sich die Nebenkomponenten 1,3-Alkylmethylpyrrolidin in einer nachfolgenden Aufarbeitung, insbesondere einer nachfolgenden Destillation, besser vom Wertprodukt (1,3-Alkylmethylpyrrolidon) abtrennen lassen.

Durch das erfindungsgemäße Verfahren wird in der Regel ein Reaktionsaustrag erhalten, der in Summe weniger als 2 Gew.-%, vorzugsweise weniger als 1 Gew.-%, besonders bevorzugt weniger als 0,5 Gew.-% einer oder mehrerer Nebenkomponenten ausgewählt aus der Gruppe bestehend aus 4-Carboxy-1-methylpyrrolidon, 4-Carbamid-1-methylpyrrolidon, 4-Hydroxymethyl-1-methylpyrrolidon und 4-Methylamino-methyl-1-methylpyrrolidon enthält.

Der Reaktionsaustrag enthält eine Mischung von 1,3- Alkylmethylpyrrolidonen der Formel (VI) und 1,4-Alkylmethylpyrrolidonen der Formel (VII).

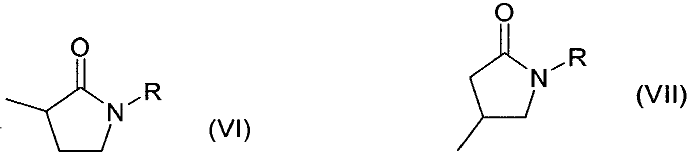

Das molare Verhältnis von 1,3- Alkylmethylpyrrolidon zu 1,4-Alkylmethylpyrrolidon im Reaktionsaustrag liegt vorzugsweise im Bereich von 1:1 bis 10:1, besonders bevorzugt 1,2:1 bis 5:1, insbesondere bevorzugt 1,3:1 bis 4:1.

Die mittels des erfindungsgemäßen Verfahrens erhältlichen Mischungen von 1,3- und 1,4-Alkylmethylpyrrolidonen können als organische Lösungsmittel, Verdünnungsmittel, Extraktionsmittel, Reinigungsmittel, Entfettungsmittel, Adsorptionsmittel und/oder Dispergierungsmittel, in der Extraktion von reinen Kohlenwasserstoffen in der petrochemischen Verarbeitung, in der Reinigung und Abtrennung von Gasen wie Acetylen 1,3-Butadien oder Isopren, in der Aromatenextraktion, beispielsweise im Distapex-Verfahren der LURGI GmbH, in der Sauergaswäsche und in der Schmierölextraktion eingesetzt werden. Die Mischungen von 1,3- und 1,4-Alkylmethylpyrrolidonen können weiterhin als Lösungsmittel für viele Kunststoffe wie Polyvinylchlorid (PVC), Polyurethane (PU), Acrylate oder Butadien-Acrylnitril-Copolymere und deren Verarbeitung eingesetzt werden. Weiterhin können sie als Reinigungsmittel bei der Entfernung von Farb- und Lackresten, sowie als Abbeizer und als Reinigungsmittel für Metall-, Keramik-, Glas- und Kunststoffoberflächen eingesetzt werden.

Ebenso können die mittels des erfindungsgemäßen Verfahren erhältlichen Mischungen von 1,3- und 1,4-Alkylmethylpyrrolidonen als Lösungsmittel oder Cosolvens für die Formulierung von Wirkstoffen im Pflanzenschutz verwendet werden.
Die mittels des erfindungsgemäßen Verfahren erhältlichen Mischungen von 1,3- und 1,4-Alkylmethylpyrrolidonen können NMP in vielen Anwendungen ersetzen.

Der Aufreinigung des Reaktionsaustrags, beispielsweise die Abtrennung des eingesetzten Lösungsmittels oder von unerwünschten Nebenprodukten, kann gemäß einem dem Fachmann bekannten Verfahren erfolgen, beispielsweise durch Destillation oder Rektifikation.

Die Vorteile der vorliegenden Erfindung bestehen darin, dass stabile Mischungen enthaltend Itaconsäure bzw. ein Itaconsäurederivat mit primären Aminen entwickelt werden konnten, die nur einen geringen Anteil an unerwünschten Nebenprodukten, insbesondere von 4-Carboxypyrrolidon, Derivate der 4-Carboxypyrrolidone der Formel (II) oder 4-Carbamidopyrrolidon, enthalten.
Speziell bei der Herstellung von 1,3-Dimethylpryrrolidon und/oder 1,4-Dimethylpyrrolidon wird die Bildung von toxischem N-Methylpyrrolidon weitestgehend vermieden. N-Methylpyrrolidon kann beispielsweise durch Folgereaktionen (Decarboxylierung/Hydrierung) aus 4-Carboxypyrrolidon entstehen.
Die Mischungen sind bei Umgebungstemperatur stabil und können ohne erhöhten technischen Aufwand gelagert und transportiert werden.
In diesen Mischungen wird die Umwandlung von Itaconsäure bzw. den Itaconsäurederivaten in die genannten Nebenprodukte weitestgehend vermieden, so dass die Itaconsäure bzw. deren Derivate in einer nachfolgenden Hydrierung nahezu vollständig zu den gewünschten 1,3- und 1,4-Alkylmethylpyrrolidone reagieren können. Somit eignen sich die erfindungsgemäßen Mischungen zur Verwendung in einem Verfahren zur Herstellung von 1,3- und 1,4-Alkylmethylpyrrolidonen, in dem die 1,3- und 1,4-Alkylmethylpyrrolidone in hoher Ausbeute und Selektivität bezogen auf die eingesetzte Itaconsäure bzw. deren Derivate erhalten werden.
Das erfindungsgemäße Verfahren ermöglicht die Herstellung von 1,3- und 1,4-Alkylmethylpyrrolidonen aus Itaconsäure bzw. deren Derivaten, ohne dass die Itaconsäure in einer separaten, gesonderten Reaktionsstufe zur 2-Methylbernsteinsäure hydriert werden müsste. Da die Herstellung der Mischungen technisch einfach zu realisieren ist, weist das erfindungsgemäße Verfahren zur Herstellung von 1,3- und 1,4-Alkylmethylpyrrolidonen eine hohe Verfahrensökonomie auf. Ein weiterer Vorteil dieser Erfindung ist, dass zur Herstellung der Mischungen Itaconsäure bzw. Itaconsäurederivate eingesetzt werden können, die auf Basis von nachwachsenden Rohstoffen hergestellt werden. Das Zurückgreifen auf nachwachsende Rohstoffe kann zu einer Schonung von erschöpfbaren Ressourcen beitragen und ermöglich ein nachhaltiges Wirtschaften.

Das erfindungsgemäße Verfahren wird anhand der nachfolgend aufgeführten Beispiele näher ausgeführt.

### Beispiele:

### Analytik

Die Reaktionsausträge und Destillate wurden gaschromatographisch analysiert, optional unter Verwendung einer definierten Menge eines internen Standards (Diethylenglykoldimethylether). Die Mischungen wurden unverdünnt in den GC-Chromatographen (Firma HP, Trägergas: Wasserstoff) auf eine 30 m DB1-Säule (Firma J+W) gespritzt und bei Ofentemperaturen von 60°C bis 300°C (Aufheizrate 8 Kelvin pro Minute bis 220°C, dann 20 Kelvin pro Minute bis 300°C) mit einem Flammenionisationsdetektor (Temperatur: 290°C) analysiert. Die Zusammensetzung wurde durch Integration der Signale des Chromatogramms bestimmt. Bei Verwendung eines internen Standards wurde die Signalintensität in die Massenanteile unter Zuhilfenahme einer zuvor durchgeführten Kalibrierung mit dem internen Standard (Diethylenglykoldimethylether) umgerechnet.

Die Reaktionsmischungen wurden außerdem durch Kernresonanzspektroskopie analysiert. Dazu wurden die Mischungen entweder in einem deuterierten Lösungsmittel (D₂O) verdünnt oder unverdünnt unter Verwendung eines externen Standards im Spektrometer (Bruker DPX400) untersucht. Die Mengenverhältnisse der Komponenten wurden durch Integration der Signale im 1 H-Spektrum bestimmt.

Die Bestimmung des Wassergehalts wurde durch Karl-Fischer-Titration durchgeführt. Dazu wurden 1-3 ml der Probenlösung in einen Automaten zur Bestimmung des Wassergehalts nach der Karl-Fischer-Methode eingespritzt (Metrohm Karl Fischer Coulometer KF756). Die Messung erfolgte auf coulometrischem Weg und basiert auf der Karl-Fischer-Reaktion,der wasservermittelten Umsetzung von lod mit Schwefeldioxid.

### Beispiel 1

Itaconsäure (1,9 kg) wurde unter Eiskühlung in 40%iger wässrige Methylaminlösung (2,6 I, 2,3 kg) eingetragen. Die homogene Lösung wurde in einen 9 Liter Autoklaven aus Edelstahl mit Rührer überführt und mit dem Katalysator Rhodium(5%)/Aktivkohle (98 g, feucht (enthält ca. 50% Wasser)) [Herkunft: AlfaAesar] versetzt. Der Autoklav wurde verschlossen und 100 bar Wasserstoffdruck wurden bei Umgebungstemperatur aufgepresst. Der Ansatz wurde 2 Stunden unter diesen Bedingungen gerührt. Anschließend wurde der Ansatz auf 200 °C aufgeheizt und bei dieser Temperatur 65 Stunden gehalten. Wasserstoff wurde kontinuierlich nachgepresst und ein Druck von 250 bar wurde gehalten. Anschließend wurde der Autoklav abgekühlt, entspannt und entleert. Der Katalysator wurde durch Filtration entfernt und das Rohprodukt wurde gaschromatographisch analysiert. Folgende Zusammensetzung (bezogen auf die organischen Anteile, d.h. ohne Berücksichtigung von Wasser) wurde gefunden:
49% 1,3-Dimethylpyrrolidon
27% 1,4-Dimethylpyrrolidon
8% 1,3-Dimethylpyrrolidin
11 % Monomethylamin
2% Dimethylamin
1% Trimethylamin
(Rest: nicht identifiziert)

4-Carboxy-1-methylpyrrolidon, 4-Carbamid-1-methylpyrrolidon, 4-Hydroxymethyl-1-methylpyrrolidon, 4-Methylamino-methyl-1-methylpyrrolidon wurden nicht nachgewiesen.
Umsatz: 100%
Selektivität (bez. auf Itaconsäure): 88%

Das Rohprodukt wurde durch Destillation im Vakuum in einer Füllkörperkolonne (Länge: 110 cm, Durchmesser: 2 cm, Füllkörper: Raschig-Ringe, 3 mm) fraktioniert destilliert. Nach Abtrennung des Wassers und überschüssigen Amins bei Atmosphärendruck wurden die Reaktionsprodukte bei 4 hPa (Siedebereich 54-57 °C am Kolonnenkopf) destilliert. Alle Fraktionen, die >99,5% (gaschromatographisch bestimmt) der Wertprodukte enthielten, wurden vereinigt.
Es wurden 1,1 kg Dimethylpyrrolidon (Gesamtausbeute 67%, Isomerenverhältnis 1,3-Dimethylpyrrolidon zu 1,4-Dimethylpyrrolidon 2:1) erhalten.

### Beispiel 2

Itaconsäure (0,65 kg) wurde unter Eiskühlung in 40%iger wässrige Methylaminlösung (1,1 I, 0,97 kg) eingetragen. Die homogene Lösung wurde in einen 3,5 Liter Autoklaven aus Edelstahl mit Rührer überführt und mit dem Katalysator Rhodium(5%)/Aktivkohle (33 g, trocken) [Herkunft: AlfaAesar] versetzt. Der Autoklav wurde verschlossen und 50 bar Wasserstoffdruck wurden bei Umgebungstemperatur aufgepresst. Anschließend wurde der Ansatz auf 200 °C aufgeheizt und bei dieser Temperatur 72 Stunden gehalten. Wasserstoff wurde kontinuierlich nachgepresst und ein Druck von 250 bar wurde gehalten. Anschließend wurde der Autoklav abgekühlt, entspannt und entleert. Der Katalysator wurde durch Filtration entfernt und das Rohprodukt wurde gaschromatographisch analysiert. Folgende Zusammensetzung (bezogen auf die organischen Anteile, d.h. ohne Berücksichtigung von Wasser) wurde gefunden:
52% 1,3-Dimethylpyrrolidon
25% 1,4-Dimethylpyrrolidon
4% 3-Methylpyrrolidon
4% 4-Methylpyrrolidon
2% 1,3-Dimethylpyrrolidin
2% Monomethylamin
1 % Dimethylamin
4% Trimethylamin
2% Methylbernsteinsäuredi(methylamid)
(Rest: nicht identifiziert)

4-Carboxy-1-methylpyrrolidon, 4-Carbamid-1-methylpyrrolidon, 4-Hydroxymethyl-1-methylpyrrolidon und 4-Methylamino-methyl-1-methylpyrrolidon wurden nicht nachgewiesen.
Umsatz: 100%
Selektivität (bez. auf Itaconsäure): 83%

### Beispiel 3

Itaconsäure (0,65 kg) wurde unter Eiskühlung in 40%iger wässrige Methylaminlösung (1,1 I, 0,97 kg) eingetragen. Die homogene Lösung wurde in einen 3,5 Liter Autoklaven aus Edelstahl mit Rührer überführt und mit dem Katalysator Rhodium(5%)/Aktivkohle (33 g, trocken) [Herkunft: AlfaAesar] versetzt. Der Autoklav wurde verschlossen und 50 bar Wasserstoffdruck wurden bei Umgebungstemperatur aufgepresst. Anschließend wurde der Ansatz auf 220 °C aufgeheizt und bei dieser Temperatur 72 Stunden gehalten. Wasserstoff wurde kontinuierlich nachgepresst und ein Druck von 250 bar wurde gehalten. Anschließend wurde der Autoklav abgekühlt, entspannt und entleert. Der Katalysator wurde durch Filtration entfernt und das Rohprodukt wurde gaschromatographisch analysiert. Folgende Zusammensetzung (bezogen auf die organischen Anteile, d.h. ohne Berücksichtigung von Wasser) wurde gefunden:
33% 1,3-Dimethylpyrrolidon
15% 1,4-Dimethylpyrrolidon
6% 3-Methylpyrrolidon
3% 4-Methylpyrrolidon
1 % 1,3-Dimethylpyrrolidin
3% Monomethylamin
4% Dimethylamin
13% Trimethylamin
9% Methylbernsteinsäuredi(methylamid)
(Rest: nicht identifiziert)

4-Carboxy-1-methylpyrrolidon, 4-Carbamid-1-methylpyrrolidon, 4-Hydroxymethyl-1-methylpyrrolidon und 4-Methylamino-methyl-1-methylpyrrolidon wurden nicht nachgewiesen.
Umsatz: 100%
Selektivität (bez. auf Itaconsäure): 60%

### Beispiel 4

Itaconsäure (2,0 kg) wurde unter Eiskühlung in 40%ige wässrige Methylaminlösung (2,6 I, 2,3 kg) eingetragen. Die homogene Lösung wurde in einen 9 Liter Autoklaven aus Edelstahl mit Rührer überführt und mit bereits für die Synthese von DMP gebrauchtem, nach Reaktionsende abfiltriertem Katalysator Rhodium(5%)/Aktivkohle (98 g, feucht, ca. 50% Wasser) [Herkunft: AlfaAesar] versetzt. Der Autoklav wurde verschlossen und 100 bar Wasserstoffdruck wurden bei Umgebungstemperatur aufgepresst. Nach 2 Stunden wurde der Ansatz auf 200 °C aufgeheizt und bei dieser Temperatur 65 Stunden gehalten. Wasserstoff wurde kontinuierlich nachgepresst und ein Druck von 200 bar wurde gehalten. Anschließend wurde der Autoklav abgekühlt, entspannt und entleert. Der Katalysator wurde durch Filtration entfernt und das Rohprodukt wurde gaschromatographisch analysiert. Folgende Zusammensetzung (bezogen auf die organischen Anteile, d.h. ohne Berücksichtigung von Wasser) wurde gefunden:
54% 1,3-Dimethylpyrrolidon
29% 1,4-Dimethylpyrrolidon
1 % 3-Methylpyrrolidon
7% 1,3-Dimethylpyrrolidin
6% Monomethylamin
2% Dimethylamin
1 % Trimethylamin
(Rest: nicht identifiziert)

4-Carboxy-1-methylpyrrolidon, 4-Carbamid-1-methylpyrrolidon, 4-Hydroxymethyl-1-methylpyrrolidon und 4-Methylamino-methyl-1-methylpyrrolidon wurden nicht nachgewiesen.
Umsatz: 100%
Selektivität (bez. auf Itaconsäure): 91%

### Beispiel 5

Itaconsäure (26 g) wurde unter Eiskühlung in 40%iger wässrige Methylaminlösung (18 ml, 16 g) eingetragen und mit Wasser (23 ml) verdünnt. Die homogene Lösung wurde in einen 300 Milliliter Autoklaven aus Edelstahl mit Rührer überführt und mit dem Katalysator Ruthenium(5%)/Aktivkohle (1,3 g, trocken) [Herkunft: AlfaAesar] versetzt. Der Autoklav wurde verschlossen und 50 bar Wasserstoffdruck wurden bei Umgebungstemperatur aufgepresst. Anschließend wurde der Ansatz auf 200 °C aufgeheizt und bei dieser Temperatur 24 Stunden gehalten. Wasserstoff wurde kontinuierlich nachgepresst und ein Druck von 200 bar wurde gehalten. Anschließend wurde der Autoklav abgekühlt, entspannt und entleert. Der Katalysator wurde durch Filtration entfernt und das Rohprodukt wurde gaschromatographisch analysiert. Folgende Zusammensetzung (bezogen auf die organischen Anteile, d.h. ohne Berücksichtigung von Wasser) wurde gefunden:
15% 1,3-Dimethylpyrrolidon
7% 1,4-Dimethylpyrrolidon
5% 3-Methylpyrrolidon
3% 4-Methylpyrrolidon
1 % Methylbernsteinsäuredi(methylamid)
(Rest: nicht identifiziert)

4-Carboxy-1-methylpyrrolidon, 4-Carbamid-1-methylpyrrolidon, 4-Hydroxymethyl-1-methylpyrrolidon und 4-Methylamino-methyl-1-methylpyrrolidon wurden nicht nachgewiesen.
Umsatz: 100%
Selektivität (bez. auf Itaconsäure): 21%

### Beispiel 6

Itaconsäure (52 g) wurde unter Eiskühlung in 40%iger wässrige Methylaminlösung (87 ml, 78 g) eingetragen. Die homogene Lösung wurde in einen 300 Milliliter Autoklaven aus Edelstahl mit Rührer überführt und mit dem Katalysator Rhodium(5%)/Aktivkohle (2,6 g, trocken) [Herkunft: AlfaAesar] versetzt. Der Autoklav wurde verschlossen und 100 bar Wasserstoffdruck wurden bei Umgebungstemperatur aufgepresst. Anschließend wurde der Ansatz auf 200 °C aufgeheizt und bei dieser Temperatur 65 Stunden gehalten. Wasserstoff wurde kontinuierlich nachgepresst und ein Druck von 200 bar wurde gehalten. Anschließend wurde der Autoklav abgekühlt, entspannt und entleert. Der Katalysator wurde durch Filtration entfernt und das Rohprodukt wurde gaschromatographisch analysiert. Folgende Zusammensetzung (bezogen auf die organischen Anteile, d.h. ohne Berücksichtigung von Wasser) wurde gefunden:
55% 1,3-Dimethylpyrrolidon
23% 1,4-Dimethylpyrrolidon
7% 1,3-Dimethylpyrrolidin
3% Monomethylamin
2% Dimethylamin
6% Trimethylamin
3% 3-Methylpyrrolidon
1 % 4-Methylpyrrolidon
(Rest: nicht identifiziert)

4-Carboxy-1-methylpyrrolidon, 4-Carbamid-1-methylpyrrolidon, 4-Hydroxymethyl-1-methylpyrrolidon und 4-Methylamino-methyl-1-methylpyrrolidon wurden nicht nachgewiesen.
Umsatz: 100%
Selektivität (bez. auf Itaconsäure): 88%

### Vergleichsbeispiel 1

Durchführung wie Beispiel 6, allerdings wurde die Reaktion während der gesamten Laufzeit der Hydrierreaktion bei 100 °C gehalten.
Folgende Zusammensetzung (bezogen auf die organischen Anteile, d.h. ohne Berücksichtigung von Wasser) wurde gefunden:
0% 1,3-Dimethylpyrrolidon
0% 1,4-Dimethylpyrrolidon
7% 1,3-Dimethylsuccinimid
34% Monomethylamin
8% Trimethylamin
35% Methylbernsteinsäure
3% 4-Carboxy-1-methylpyrrolidon
7% Methylbernsteinsäuredi(methylamid)
(Rest: nicht identifiziert)

Umsatz: 100%
Selektivität (bez. auf Itaconsäure): 0%

### Vergleichsbeispiel 2

Durchführung wie Beispiel 6, allerdings wurde als Ausgangsmischung eine Lösung von 4-Carboxy-1-methylpyrrolidon (20 g), die durch Inkontaktbringen von Methylamin und Itaconsäure bei Temperaturen von mehr als 100°C erhalten wurde (hergestellt in Analogie zu Vergleichsbeispiel 4) in Wasser (80 g) sowie Rhodium(5%)/Aktivkohle (1 g) eingesetzt.
Folgende Zusammensetzung (bezogen auf die organischen Anteile, d.h. ohne Berücksichtigung von Wasser) wurde nach 65 Stunden gefunden:
40% 1,3-Dimethylpyrrolidon
24% 1,4-Dimethylpyrrolidon
3% N-Methylpyrrolidon (NMP)
16% 1,3-Dimethylsuccinimid
2% 4-Carboxy-1-methylpyrrolidon
7% 4-Hydroxymethyl-1-methylpyrrolidon
1 % 4-Carbamid-1-methylpyrrolidon
(Rest: nicht identifiziert)

Umsatz: 98%
Selektivität (bez. auf Itaconsäure): 65%

### Beispiel 7

Itaconsäure (32 g) wurde unter Eiskühlung in 40%iger wässrige Methylaminlösung (43 ml, 39 g) eingetragen. Die homogene Lösung wurde in einen 100 Milliliter Autoklaven aus Edelstahl mit Rührer überführt und mit dem Katalysator Rhodium(5%)/Aktivkohle (1,6 g, trocken, 5 Gew% bez. auf Itaconsäure) [Herkunft: AlfaAesar] versetzt. Der Autoklav wurde verschlossen und 50 bar Wasserstoffdruck wurden bei Umgebungstemperatur aufgepresst. Anschließend wurde der Ansatz auf 200 °C aufgeheizt und bei dieser Temperatur 24 Stunden gehalten. Wasserstoff wurde kontinuierlich nachgepresst und ein Druck von 200 bar wurde gehalten. Anschließend wurde der Autoklav abgekühlt, entspannt und entleert. Der Katalysator wurde durch Filtration entfernt und das Rohprodukt wurde gaschromatographisch analysiert. Folgende Zusammensetzung (bezogen auf die organischen Anteile, d.h. ohne Berücksichtigung von Wasser) wurde gefunden:
49% 1,3-Dimethylpyrrolidon
18% 1,4-Dimethylpyrrolidon
8% 1,3-Dimethylsuccinimid
1 % 1,3-Dimethylpyrrolidin
4% Monomethylamin
1 % Dimethylamin
<1% Trimethylamin
1 % 3-Methylpyrrolidon
1 % 4-Methylpyrrolidon
(Rest: nicht identifiziert)

4-Carboxy-1-methylpyrrolidon, 4-Carbamid-1-methylpyrrolidon, 4-Hydroxymethyl-1-methylpyrrolidon und 4-Methylamino-methyl-1-methylpyrrolidon wurden nicht nachgewiesen.
Umsatz: 100%
Selektivität (bez. auf Itaconsäure): 71%

### Beispiel 8 (Herstellung der Zulaufmischung bei 50°C)

Itaconsäure (18,5 g; 0,142 mol) wurde unter Wasserbadkühlung langsam und portionsweise zu einer wässrigen Lösung von Methylamin (41% in Wasser, 21,5 g bez. auf die wässr. Lösung, 0,284 mol, 2,0 Äquivalente) gegeben, so dass die Temperatur während des Mischvorgangs stets unter 50 °C blieb. Die Mischung wurde anschließend 1 Stunde bei 50 °C gerührt. Die Zusammensetzung der Mischung wurde per 1H-NMR (Lösungsmittel D₂O als externer Standard) anhand der olefinischen Signale der Itaconsäure (als Ammoniumsalz vorliegend) (chemische Verschiebung 5,3 ppm und 5,7 ppm) und der Signale der Methylen-Funktion neben dem Stickstoffatom des 4-Carboxy-1-methylpyrrolidon (chemische Verschiebung 3,6-3,8 ppm) analysiert. Das Verhältnis Itaconsäure : 4-Carboxy-1-methylpyrrolidon betrug 100 : 0.

### Beispiel 9 (Herstellung der Zulaufmischung bei 100 °C)

Itaconsäure und Methylamin wurden wie in Beispiel 8 beschrieben gemischt und anschließend für 1 Stunde auf 100 °C erhitzt. NMR-Untersuchung ergab, dass das Verhältnis Itaconsäure : 4-Carboxy-1-methylpyrrolidon 76 : 24 betrug.

### Vergleichsbeispiel 3 (Herstellung der Zulaufmischung bei 150 °C)

Itaconsäure und Methylamin wurden wie in Beispiel 8 beschrieben gemischt und anschließend für 1 Stunde auf 150 °C erhitzt. NMR-Untersuchung ergab, dass das Verhältnis Itaconsäure : 4-Carboxy-1-methylpyrrolidon 0 : 100 betrug.

### Beispiel 10 (Herstellung der Zulaufmischung Itaconsäure/Methylamin 1:1 bei 50 °C)

Itaconsäure (18,5 g; 0,142 mol) und Methylamin (41% in Wasser, 10,7 g bez. auf die wässr. Lösung, 0,142 mol, 1,0 Äquivalente) wurden wie in Beispiel 8 gemischt. Zusätzlich war die Zugabe von 20 ml Wasser erforderlich, um Niederschlagsbildung bei 50 °C zu vermeiden. NMR-Untersuchung ergab, dass das Verhältnis Itaconsäure : 4-Carboxy-1-methylpyrrolidon 100 : 0 betrug.

### Vergleichsbeispiel 4 (Herstellung der Zulaufmischung Itaconsäure/Methylamin 1:1 bei Siedetemperatur)

Itaconsäure (100 g, 0,78 mol) und Methylamin (41% in Wasser, 60,0 g bez. auf die wässr. Lösung, 0,80 mol, 1,0 Äquivalente) wurden wie in Beispiel 8 gemischt. Die Suspension wurde 1 Stunde bei Normaldruck unter Rückfluss zum Sieden erhitzt (Sdp. steigt dabei auf ca. 115 °C an). Die Feststoffe lösten sich dabei vollständig. Bei Abkühlung auf Umgebungstemperatur bildete sich ein Niederschlag. NMR-Untersuchung der homogenen Lösung und der ausgefallenen Kristalle (Lösungsmittel D₂O) ergab, dass das Verhältnis Itaconsäure : 4-Carboxy-1-methylpyrrolidon in beiden Phasen 0 : 100 betrug.
Das Produkt 4-Carboxy-1-methylpyrrolidon konnte durch Umkristallisation aus Ethylacetat gereinigt werden und wurde in Form farbloser Kristalle erhalten (Ausbeute: 59 g, 67%).

## Patentansprüche

1. Verwendung von Mischungen enthaltend Itaconsäure oder ein Itaconsäurederivat und ein primäres Amin der Formel (I),
R-NH₂ (I)
wobei man als Itaconsäurederivat einen Itaconsäure-Ester, ein Itaconsäure-Amid, ein Itaconsäure-Halogenid, ein Salz der Itaconsäure oder ein Itaconsäure-Imin einsetzt und das molare Verhältnis von primärem Amin zu Itaconsäure oder dem Itaconsäurederivat im Bereich von 0,5:1 bis 20:1 liegt, **dadurch gekennzeichnet, dass** die Mischung 50 Molprozent oder weniger 4-Carboxypyrrolidone der Formel (II), Derivate der 4-Carboxypyrrolidone der Formel (IX) und 4-Carbamidopyrrolidone der Formel (III) bezogen auf die eingesetzte Itaconsäure oder das eingesetzte Itaconsäurederivat enthält, 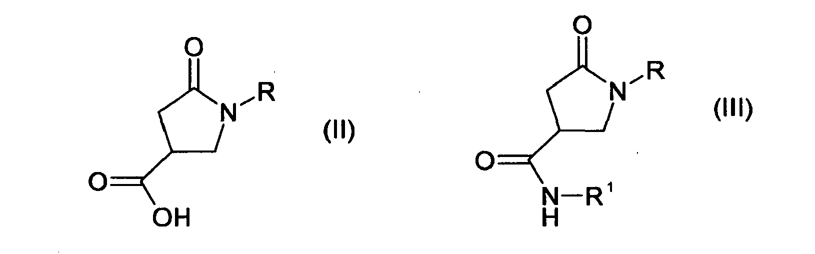 und in der R einen linearen oder verzweigten gesättigten aliphatischen Rest mit 1 bis 24 C-Atomen oder einen gesättigten cycloaliphatischen Rest mit 3 bis 24 C-Atomen bedeutet 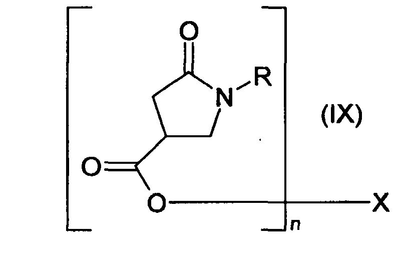 wobei n eine ganze Zahl von 1 bis 4
und X entweder H, R, ein Metall M oder NH₃R bedeutet, wobei R die voranstehend genannte Bedeutung hat,
zur Herstellung von 1,3- Alkylmethylpyrrolidonen der allgemeinen Formel (VI) und/oder 1,4-Alkylmethylpyrrolidonen allgemeinen Formel (VII) 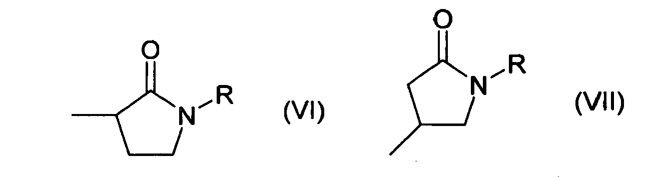 wobei R die oben genannte Bedeutung hat.

2. Verfahren zur Herstellung von 1,3-Alkylmethylpyrrolidonen der allgemeinen Formel (VI) und/oder 1,4-Alkylmethylpyrrolidonen der allgemeinen Formel (VII) 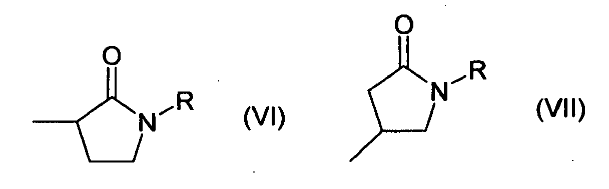 durch Umsetzung einer Mischung enthaltend Itaconsäure oder ein Itaconsäurederivat und ein primäres Amin der Formel (I) mit Wasserstoff in Gegenwart eines Hydrierkatalysators,
R-NH₂ (I)
wobei man als Itaconsäurederivat einen Itaconsäure-Ester, ein Itaconsäure-Amid, ein Itaconsäure-Halogenid, ein Salz der Itaconsäure oder ein Itaconsäure-Imin einsetzt und das molare Verhältnis von primärem Amin zu Itaconsäure oder dem Itaconsäurederivat im Bereich von 0,5:1 bis 20:1 liegt und die Mischung 50 Molprozent oder weniger 4-Carboxypyrrolidone der Formel (II), Derivate der 4-Carboxy-pyrrolidone der Formel (IX) und 4-Carbamidopyrrolidone der Formel (III) bezogen auf Itaconsäure oder Itaconsäurederivat enthält, 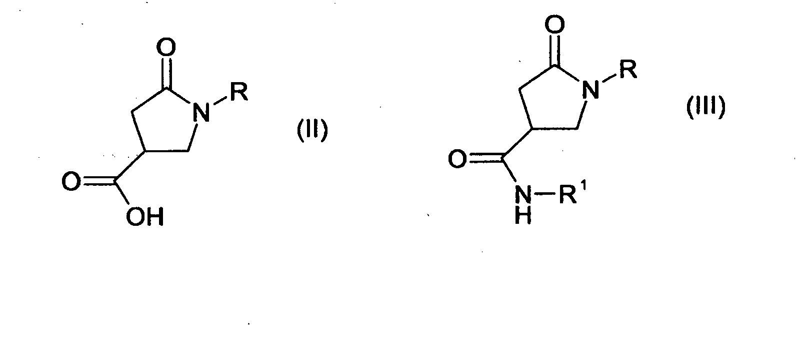 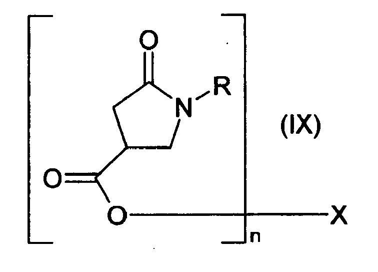 und in der R, X und n die in Anspruch 1 genannten Bedeutungen haben.

3. Verfahren zur Herstellung von Alkylmethylpyrrolidonen gemäß Anspruch 2, **dadurch gekennzeichnet, dass** die Temperatur für die Hydrierung in einem Bereich von 100 bis 300°C und der Druck in einem Bereich von 50 bis 300 bar liegt.

4. Verfahren zur Herstellung von Alkylmethylpyrrolidonen gemäß mindestens einem der Ansprüche 2 bis 3, **dadurch gekennzeichnet, dass** der Hydrierkatalysator Rhodium, Ruthenium oder Cobalt enthält.

5. Verfahren zur Herstellung von Alkylmethylpyrrolidonen gemäß mindestens einem der Ansprüche 2 bis 4, **dadurch gekennzeichnet, dass** die Produktmischung weniger als 1 % N-Methylpyrrolidon als Nebenprodukt enthält.

6. Verfahren zur Herstellung von Alkylmethylpyrrolidonen gemäß mindestens einem der Ansprüche 2 bis 5, **dadurch gekennzeichnet, dass** der Umsatz bezogen auf die eingesetzte Itaconsäure bzw. das eingesetzte Itaconsäurederivat 99% und mehr beträgt.

## Claims

1. The use of mixtures comprising itaconic acid or an itaconic acid derivative and a primary amine of the formula (I)
R-NH₂ (I)
where the itaconic acid derivative used is an itaconic ester, an itaconamide, an itaconyl halide, a salt of itaconic acid or an itaconimine and the molar ratio of primary amine to itaconic acid or the itaconic acid derivative is in the range from 0.5:1 to 20:1, wherein the mixture comprises 50 mole percent or less of 4-carboxypyrrolidones of the formula (II), derivatives of the 4-carboxypyrrolidones of the formula (IX) and 4-carbamidopyrrolidones of the formula (III), based on the itaconic acid used or the itaconic acid derivative used, 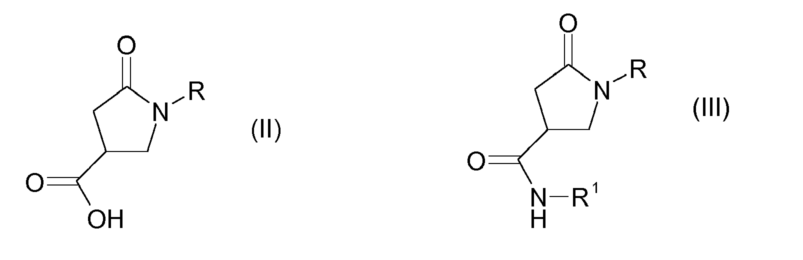 and in which R is a linear or branched saturated aliphatic radical having 1 to 24 carbon atoms or a saturated cycloaliphatic radical having 3 to 24 carbon atoms, 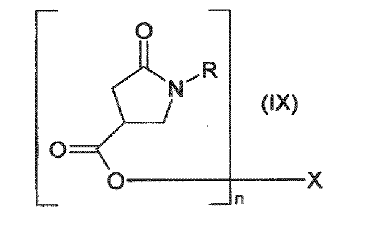 where n is an integer from 1 to 4
and X is H, R, a metal M or NH₃R, where R is as defined above, and for preparing 1,3-alkylmethylpyrrolidones of the general formula (VI) and/or 1,4-alkylmethylpyrrolidones of the general formula (VII) 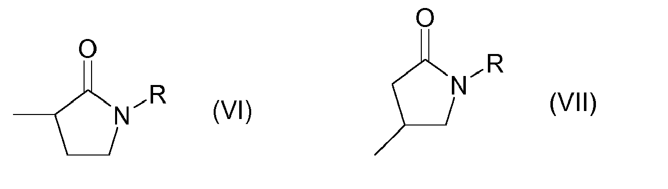 where R is as defined above.

2. A process for preparing 1,3-alkylmethylpyrrolidones of the general formula (VI) and/or 1,4-alkylmethylpyrrolidones of the general formula (VII) 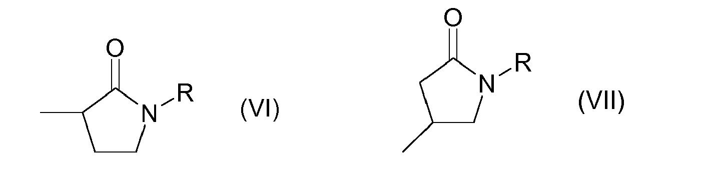 by reacting a mixture comprising itaconic acid or an itaconic acid derivative and a primary amine of the formula (I) with hydrogen in the presence of a hydrogenation catalyst
R-NH₂ (I)
where the itaconic acid derivative used is an itaconic ester, an itaconamide, an itaconyl halide, a salt of itaconic acid or an itaconimine and the molar ratio of primary amine to itaconic acid or the itaconic acid derivative is in the range from 0.5:1 to 20:1 and the mixture comprises 50 mole percent or less of 4-carboxypyrrolidones of the formula (II), derivatives of the 4-carboxypyrrolidones of the formula (IX) and 4-carbamidopyrrolidones of the formula (III), based on itaconic acid or itaconic acid derivative, 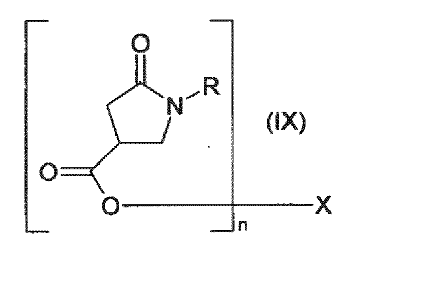 and in which R, X and n are as defined in claim 1.

3. The process for preparing alkylmethylpyrrolidones according to claim 2, wherein the temperature for the hydrogenation is within a range from 100 to 300°C and the pressure is within a range from 50 to 300 bar.

4. The process for preparing alkylmethylpyrrolidones according to at least one of claims 2 to 3, wherein the hydrogenation catalyst comprises rhodium, ruthenium or cobalt.

5. The process for preparing alkylmethylpyrrolidones according to at least one of claims 2 to 4, wherein the product mixture comprises less than 1% N-methylpyrrolidone as a by-product.

6. The process for preparing alkylmethylpyrrolidones according to at least one of claims 2 to 5, wherein the conversion based on the itaconic acid used or the itaconic acid derivative used is 99% and more.

## Revendications

1. Utilisation de mélanges contenant de l'acide itaconique ou un dérivé de l'acide itaconique et une amine primaire de formule (I)
**R-NH₂** (I)
en utilisant, comme dérivé de l'acide itaconique, un ester de l'acide itaconique, un amide de l'acide itaconique, un halogénure de l'acide itaconique, un sel de l'acide itaconique ou une imine de l'acide itaconique et le rapport molaire de l'amine primaire à l'acide itaconique ou au dérivé de l'acide itaconique se situant dans la plage de 0,5:1 à 20:1, **caractérisée en ce que** le mélange contient 50% en mole ou moins de 4-carboxypyrrolidones de formule (II), des dérivés des 4-carboxypyrrolidones de formule (IX) et des 4-carbamidopyrrolidones de formule (III) par rapport à l'acide itaconique utilisé ou au dérivé de l'acide itaconique utilisé, 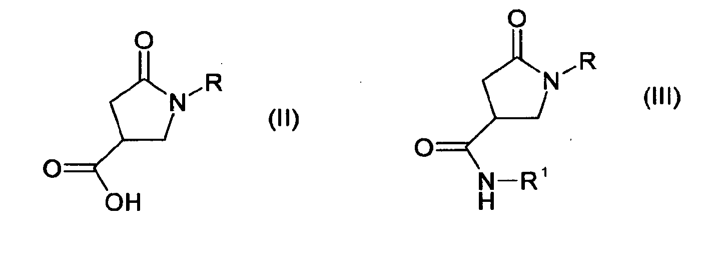 et dans lesquelles R signifie un radical aliphatique linéaire ou ramifié, saturé comprenant 1 à 24 atomes de carbone ou un radical cycloaliphatique saturé comprenant 3 à 24 atomes de carbone 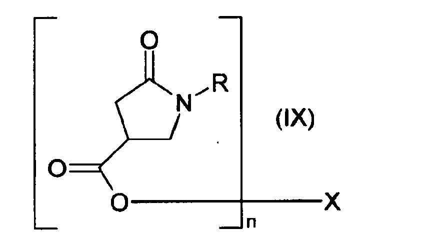 n valant un nombre entier de 1 à 4
et X signifiant H, R, un métal M ou NH₃R, R présentant la signification indiquée ci-dessus,
pour la préparation de 1,3-alkylméthylpyrrolidones de formule générale (VI) et/ou de 1,4-alkylméthylpyrrolidones de formule générale (VII) 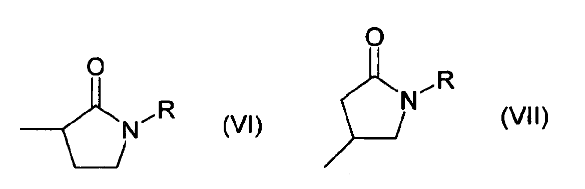 R présentant la signification susmentionnée.

2. Procédé pour la préparation de 1,3-alkylméthylpyrrolidones de formule générale (VI) et/ou de 1,4-alkylméthylpyrrolidones de formule générale (VII) 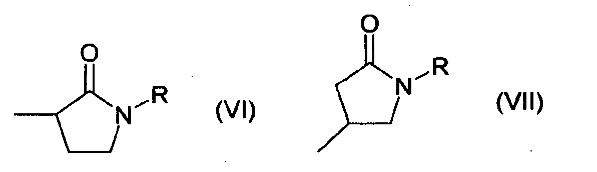 par transformation d'un mélange contenant de l'acide itaconique ou un dérivé de l'acide itaconique et une amine primaire de formule (I) avec de l'hydrogène en présence d'un catalyseur d'hydrogénation
**R-NH₂** (I)
en utilisant, comme dérivé de l'acide itaconique, un ester de l'acide itaconique, un amide de l'acide itaconique, un halogénure de l'acide itaconique, un sel de l'acide itaconique ou une imine de l'acide itaconique et le rapport molaire de l'amine primaire à l'acide itaconique ou au dérivé de l'acide itaconique se situant dans la plage de 0,5:1 à 20:1 et le mélange contenant 50% en mole ou moins de 4-carboxypyrrolidones de formule générale (II), de dérivés des 4-carboxypyrrolidones de formule (IX) et de 4-carbamidopyrrolidones de formule (III) par rapport à l'acide itaconique ou au dérivé de l'acide itaconique, 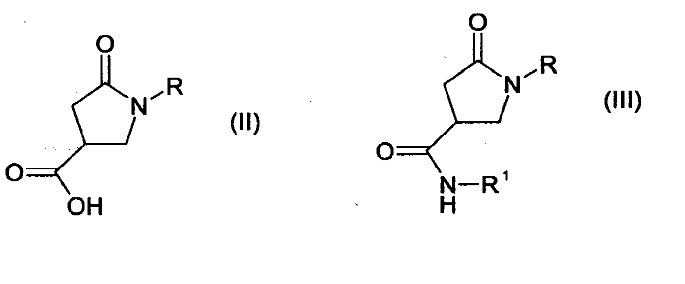 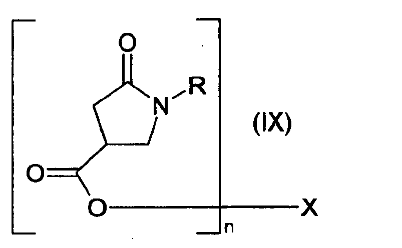 dans lesquelles R, X et n ont les significations mentionnées dans la revendication 1.

3. Procédé pour la préparation d'alkylméthylpyrrolidones selon la revendication 2, **caractérisé en ce que** la température pour l'hydrogénation se situe dans une plage de 100 à 300°C et la pression se situe dans une plage de 50 à 300 bars.

4. Procédé pour la préparation d'alkylméthylpyrrolidones selon au moins l'une quelconque des revendications 2 à 3, **caractérisé en ce que** le catalyseur d'hydrogénation contient du rhodium, du ruthénium ou du cobalt.

5. Procédé pour la préparation d'alkylméthylpyrrolidones selon au moins l'une quelconque des revendications 2 à 4, **caractérisé en ce que** le mélange de produits contient moins de 1% de N-méthylpyrrolidone comme produit secondaire.

6. Procédé pour la préparation d'alkylméthylpyrrolidones selon au moins l'une quelconque des revendications 2 à 5, **caractérisé en ce que** la conversion, par rapport à l'acide itaconique utilisé ou au dérivé de l'acide itaconique utilisé, est de 99% et plus.
